(19) Europäisches Patentamt
European Patent Office
Office européen des brevets

(11) Veröffentlichungsnummer : **0 398 024 B1**

(12) # EUROPÄISCHE PATENTSCHRIFT

(45) Veröffentlichungstag der Patentschrift : **24.02.93 Patentblatt 93/08**

(51) Int. Cl.$^5$ : **A61K 49/02,** A61K 47/48, A61K 49/00, A61K 43/00

(21) Anmeldenummer : **90107187.8**

(22) Anmeldetag : **14.03.90**

(54) **Konjugate zur Tumorlokalisation und/oder Tumortherapie.**

(30) Priorität : **19.04.89 DE 3912792**

(43) Veröffentlichungstag der Anmeldung : **22.11.90 Patentblatt 90/47**

(45) Bekanntmachung des Hinweises auf die Patenterteilung : **24.02.93 Patentblatt 93/08**

(84) Benannte Vertragsstaaten : **AT BE CH DE DK ES FR GB GR IT LI LU NL SE**

(56) Entgegenhaltungen :
**US-A- 4 466 951**
**NUCL. MED: BIOL., Band 15, Nr. 5, 1988, Pergamon Press plc, Oxford (GB); S.A.ALI et al., Seiten 557-561.**
**BIOCHEM. J., vol. 212, 1983, London (GB); R.C.PITTMAN et al., Seiten 791-800.**

(73) Patentinhaber : **Deutsches Krebsforschungszentrum Stiftung des öffentlichen Rechts**
**Im Neuenheimer Feld 280**
**W-6900 Heidelberg 1 (DE)**

(72) Erfinder : **Sinn, Hansjörg Dr.**
**Ahornweg 10**
**W-6908 Wiesloch (DE)**
Erfinder : **Schrenk, Hans-Hermann**
**Mittelgasse 3**
**W-6721 Zeiskam (DE)**
Erfinder : **Maier-Borst, Wolfgang, Dr.**
**Schlüsselweg 9**
**W-6901 Dossenheim (DE)**
Erfinder : **Friedrich, Eckhard, Dr.**
**In den Hofwiesen 6**
**W-6741 Ilbesheim (DE)**
Erfinder : **Graschew, Georgi, Dr.**
**Im Langgewann 5/29**
**W-6900 Heidelberg (DE)**
Erfinder : **Wöhrle, Dieter, Prof. Dr.**
**Lothringer Strasse 29**
**W-2800 Bremen (DE)**

(74) Vertreter : **Müller-Boré & Partner Patentanwälte**
**Isartorplatz 6 Postfach 26 02 47**
**W-8000 München 2 (DE)**

**EP 0 398 024 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Konjugate, die aufgrund ihrer Zusammensetzung eine erhöhte biologische Halbwertszeit aufweisen und sich bevorzugt im Tumorgewebe anreichern. Diese Konjugate sind dazu geeignet, zum einen eine sehr empfindliche nuklearmedizinische Tumordiagnostik zu ermöglichen und zum anderen beispielsweise in der Röntgendiagnostik, Computertomographie, Kernspintomographie, Electron-Spin-Resonance-Spektroskopie, oder Elektronenmikroskopie auch neue Tumorerkennungsverfahren zu bieten. Durch die Integration photoaktiver Bausteine in die erfindungsgemäßen Konjugate eignen sich diese außerdem zur Laser-Fluoreszenz-Diagnostik und zur photodynamischen Therapie bösartiger Tumore. Weiterhin kann die hohe Tumoranreicherung der erfindungsgemäßen Konjugate zum Transport von Zytostatika zum Tumor genutzt werden.

Eine heute weitverbreitete Technik zur Lokalisation von Tumoren bedient sich der Spezifität bestimmter Antikörper gegen tumorassoziierte Antigene. Solche Antikörper, insbesondere werden hierzu monoklonale Antikörper verwendet, lassen sich relativ leicht mit den radioaktiven Nukliden des Jods direkt markieren. Die Markierung mit anderen, für die Diagnostik oder Therapie geeigneten radioaktiven Nukliden wie Indium-111, gestaltet sich etwas schwieriger, da diese meist nur über geeignete Chelatbildner (z. B. DTPA) an den Antikörper gebunden werden können. Diese Chelatisierungstechnik wird heute mehr oder weniger erfolgreich betrieben.

Die so markierten Antikörper weisen jedoch den großen Nachteil auf, daß nach erfolgter Injektion die radioaktiven Nuklide bzw. die chelatisierten radioaktiven Nuklide im Körper von dem betreffenden Antikörper abgespalten werden und somit erst gar nicht im Zielorgan landen. Insbesondere die chelatisierten Metalle (wie Indium-111-DTPA) werden in der Leber abgefangen und reichern sich dort an. Die Vermutung liegt nahe, daß der abgespaltene Indium-111-DTPA-Komplex im Abwehrsystem (retikuloendothelialem System) verbleibt. Deshalb ist es nicht möglich, Tumoranreicherungen in der Lebergegend oder im Knochenmark nachzuweisen. Es wäre wünschenswert, wenn die spezifische Akkumulation am Tumor noch weiter gesteigert werden könnte. Ein sehr wesentlicher technischer Fortschritt wäre eine Methode zur Vermeidung der Speicherung der über Chelatbildner gebundenen Radionuklide im retikuloendothelialen System. Diese beiden Entwicklungsschritte würden die Effizienz und insbesondere auch die diagnostische Sicherheit beim Tumornachweis wesentlich steigern. Sie wären aber noch wesentlich bedeutsamer für die Anwendung radioaktiv markierter Substanzen in der Therapie. Die bisher vereinzelt durchgeführten Behandlungen hatten alle den Nachteil, daß daß die Tumordosis aus Rücksicht auf die zu schonenden Organe relativ klein gewählt werden mußten. Nur eine möglichst selektive Anreicherung des transportierten Radionuklids und eine lange Verweilzeit im Tumor läßt eine hohe Strahlendosis am Tumor bei weitgehender Schonung der gesunden Gewebe möglich erscheinen.

Pittman et al. (Biochem. J. (1983), 212, 791 - 800) beschreiben Katabolismusuntersuchungen von Lipoproteinen, wobei die Lipoproteine mit einem Tyramin-Cellobiose-Label markiert sind.

Ali et al. (Nucl. Med. Biol., Vol. 15, No. 5, pp. 557 - 561, (1988)) und Walch et al. (Proc. 7th Intern. Symp. on Radiopharm. Chem., 1988, Paper 120) berichteten über den Einsatz eines Tyramin-Cellobiose-Antikörperkonjugats, der im Tyraminteil mit radioaktivem Jod markiert war. Dabei wurde zwar festgestellt, daß die prozentuale Aktivität im Tumor zunahm, die Leberspeicherung jedoch nach wie vor zu hoch war.

Maxwell et al. (J. Biol. Chem., Vol. 263, No. 28, pp. 14122 to 14127, (1988)) beschreibenen einen Inulin-$^{125}$J-Tyramin-Label für langlebige Zirkulationsproteine. Durch Ankopplung dieses Labels an Zirkulationsproteine wie Rattenserumalbumin (RSA) lassen sich dann jene Stellen im Organismus lokalisieren, an denen die Zirkulationsproteine abgebaut werden (Katabolismus der Plasmaproteine). Maxwell et al. stellten fest, daß größere Glycokonjugate, wie das beschriebene Inulin-Konjugat, besser für die Katabolismusuntersuchungen geeignet sind als kleinere Glycokonjugate, da diese großen Konjugate am Abbauort des Plasmaproteins ausfallen und nicht oder nur in geringem Umfang abtransportiert werden.

Pittmann et al. (US-A-4 466 951) beschreiben ein Konjugat aus Cellobiose, radioaktiv markiertem Tyramin, Cyanurchlorid und einem monoklonalen Antikörper. Es wird ausgedrückt, daß das Konjugat aufgrund der nichtmetabolisierbaren Cellobiose an Stellen des Organismus festgehalten wird, an denen das Protein (Antikörper) spezifisch oder unspezifisch aufgenommen bzw. abgebaut wird.

Es wurde nun überraschend gefunden, daß Konjugate, bestehend aus

a) mindestens einem Polyalkohol oder einem derivatisierten Polyalkohol
b) mindestens einem aktiven Agens,
c) mindestens einem Linker und
d) einem Protein, wobei

der oder die Polyalkohole oder der oder die derivatisierten Polyalkohole vom Abwehrsystem eines Organismus nicht als körperfremd erkannte Polyalkohole oder derivatisierte Polyalkohole sind und das Protein ein vom Tumor spezifisch oder unspezifisch aufnehmbares, vom Abwehrsystem eines Organismus nicht als körperfremd erkanntes Protein ist, die dadurch gekennzeichnet sind, daß der oder die Polyalkohole oder der

oder die derivatisierten Polyalkohole eine Verbindung der Formel I sind,

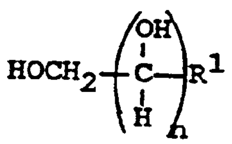 (I)

worin
$R^1$ $CH_2OH$, CHO oder $CH_2NH_2$ bedeutet und
n größer oder gleich 1 ist, bevorzugt ist n 1 - 10, besonders bevorzugt 3 - 6,
und worin eine

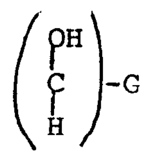-Gruppe

durch CO ersetzt sein kann und worin keine, eine oder mehrere OH-Gruppen durch $NH_2$, $^{19}F$, $C^{19}F_3$, mono- oder poly-$^{19}F$-substituiertes $C_1$-$C_4$-Alkyl oder mono-, di-, tri-, tetra- oder penta-$^{19}F$-substituiertes Phenyl ersetzt sein können, ausgezeichnet zur Tumordiagnose und/oder Tumortherapie geeignet sind.

Ebenso geeignet sind Glucose, Fructose, Maltose,oder Saccharose, wobei mindestens eine OH-Gruppe in diesen Verbindungen durch $^{19}F$, $C^{19}F_3$, mono- oder poly-$^{19}F$-substituiertes $C_1$-$C_4$Alkyl oder mono-, di-, tri-, tetra- oder penta-$^{19}F$-substituiertes Phenyl ersetzt ist.

Beispiele für solche Polyalkohole sind Glucose, Fructose, Disaccharide wie Saccharose, Maltose, sowie Sorbit und Glycerinaldehyd. Sofern die Leberspeicherung für die Diagnose bestimmter Tumore keine oder nur eine untergeordnete Rolle spielt, eignen sich auch Polyalkohole wie Galactose.

Weiterhin betrifft die Erfindung ein Verfahren zur Herstellung der erfindungsgemäßen Konjugate sowie Tumordiagnostika, Tumortherapeutika, Verfahren zur Erkennung von Tumoren und Verfahren zur Behandlung von Tumoren unter Verwendung der erfindungsgemäßen Konjugate.

Unter einem Konjugat wird eine durch chemische Bindungen zusammengehaltene Substanz, die aus mehreren aerschiedenen Molekülen mit charakteristischen Eigenschaften aufgebaut ist, verstanden. Dabei können die chemischen Bindungen ionisch, kovalent oder koordinativ sein, bevorzugt kovalent.

Die erfindungsgemäßen Konjugate weisen den Vorteil auf, daß sie vom Abwehrsystem des Organismus nicht als körperfremd angesehen werden und dementsprechend auch nicht oder nur in geringem Umfang von Organen wie der Leber abgefangen, abgebaut und ausgeschieden oder dort abgelagert werden. Dies wird erreicht durch die schützende Funktion des oder der Polyalkohole, die die meist körperfremde aktive Komponente - also das oder die aktiven Agenzien - abschirmt, so daß sie vom Abwehrsystem des Körpers nicht erkannt werden. Um nun sicherzustellen, daß die solchermaßen getarnte aktive Komponente selektiv zu Tumorgeweben transportiert wird und dort auch verbleibt, wird über einen Linker - auch Spacer genannt - ein Protein, das vom Tumor spezifisch oder unspezifisch aufgenommen wird, an diese getarnte aktive Komponente gebunden. Das Protein selbst ist nun so ausgewählt, daß es einerseits vom Abwehrsystem des Körpers nicht erkannt und andererseits spezifisch oder unspezifisch in Tumoren angereichert wird. Injiziert man nun ein solchermaßen zusammengesetztes Konjugat, so verbleibt dies so lange vom Abwehrsystem des Körpers unerkannt in der Zirkulation, bis es spezifisch oder unspezifisch zu einem hohen Prozentsatz von Tumorzellen aufgenommen wird. In den Tumorzellen wird dann das Protein entweder katabolisiert oder es bindet spezifisch an die tumorassoziierten Antigene. Wird es abgebaut, so verbleibt schließlich das um das Protein reduzierte Konjugat in der Tumorzelle, weil es aufgrund seiner tarnenden Polyalkoholschutzgruppen weiterhin nicht als körperfremd erkannt wird und dementsprechend auch nicht aus den Tumorzellen ausgeschleust wird. In beiden Fällen - Konjugat mit abbaubarem Protein und Konjugat mit antigenspezifischem Protein - akkumuliert das Konjugat im Tumor (kummulativer Label) und kann über sein aktives Agens oder seine aktiven Agenzien mittels geeigneter Methoden lokalisiert werden bzw. kann direkt am Ort des Tumors seine therapeutische Wirkung entfalten, je nach Art des aktiven Agens. Diese erfindungsgemäßen Konjugate weisen eine überraschend hohe Tumoranreicherungsrate auf, die bei Versuchstieren bei ca. 25 - 30 % der applizierten Dosis oder höher liegt, bei gleichzeitiger äußerst niedriger Anreicherungsrate im retikuloendothelialen System.

Unter einem nicht immunologisch erkennbaren Polyalkohol werden solche Polyalkohole oder Polyalkoholderivate verstanden, die vom körpereigenen Abwehrsystem nicht als körperfremd erkannt werden und infolgedessen nicht oder nur unwesentlich von den entsprechenden Organen abgefangen werden.

Unter einem aktiven Agens wird eine Verbindung verstanden, die entweder in der Lage ist, ein Signal an eine externe Abtastvorrichtung zu geben, oder die in der Lage ist, auf Tumorgewebe direkt oder indirekt therapeutisch einzuwirken oder aber beide Funktionen gleichzeitig ausüben kann. Genauso gut kann das aktive Agens bi- oder multifuntionell sein, d. h. es kann zwei oder mehrere unterschiedliche Signale aussenden, die dann von unterschiedlichen Abtastvorrichtungen aufgefangen werden.

Verbindungen, welche ein Signal an eine externe Abtastvorrichtung abgeben können, sind bevorzugt solche gemäß Formel II

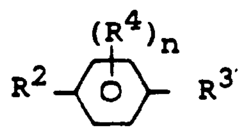

(II)

worin
$R^2$ $NH_2$, $C_1$-$C_4$-Alkylamin oder CHO bedeutet und
$R^3$ $NO_2$, $NH_2$, OH oder NCS bedeutet und
$R^4$ $^{131}J$, $^{123}J$, $^{19}F$, $C^{19}F_3$, OH,H bedeutet und
n 1 bis 4 ist.

Beispielsweise können eingesetzt werden radioaktiv markierte Substanzen wie J-123- oder J-131-markiertes Tyramin oder Cyclame (cyclische Amine), welche in der Lage sind, Metalle wie In, Ga, Gd, Sm, Y, Re, Rh, Ru oder Pt zu komplexieren.

Werden solche Verbindungen in die erfindungsgemäßen Konjugate eingebaut, so eignen sich diese Konjugate zu szintigraphischen Darstellungen mit γ-Kamera- oder SPECT-(single photon emission computer tomographie)-Systemen. Andere Verbindungen, die ein Signal an eine externe Abtastvorrichtung abgeben können, sind z. B. Fluoreszenzmarker oder aktive Agenzien, die sich für einen Einsatz in der

- Computer Tomographie (CT) eignen. Beispielsweise eignen sich hierzu hoch jodierte Fluoresceinisothio- cyanate (FITC).

Oder aktive Agenzien, die sich für einen Einsatz in der

- Kernspin Computer Tomographie (NMR) eignen. Beispiele hierzu sind fluorierte aliphatische ($-CF_3$) oder aromatische (∅-$(F)_4$) Gruppen (∅ = Phenyl).

Oder aktive Agenzien, die sich für einen Einsatz in der

- Elektronen Spin Resonanz Spektroskopie (ESR) eignen. Beispiele hierfür sind heterocyclische Ringsysteme mit Radikalcharakter wie 4-Amino-2,2,6,6-tetramethyl-1-piperidin-oxid.

Oder aktive Agenzien, die sich für einen Einsatz in der

- Neutron-capture-therapy (NCT) eignen. Beispiele hierfür sind borhaltige Verbindungen wie $Na_2{}^{10}B_{12}H_{11}$-SH oder andere, borhaltige Derivate von Porphyrinen, Phthalocyaninen, Naphthalocyaninen etc.

Externe Abtastvorrichtungen sind Vorrichtungen gemäß Stand der Technik, die die von den aktiven Agenzien ausgehenden Signale in auswertbare Informationen umwandeln. Beispiele hierfür sind γ-Kamera- oder SPECT-Systeme, die die von den radioaktiv markierten Verbindungen ausgesandten γ-Strahlen bzw. Photonen auffangen und in Bilder (Szintigramme) umsetzen.

Computertomographen sind Geräte, die die unterschiedliche Abschwächung eines den Organismus durchdringenden Röntgenstrahls registrieren und in Bildinformationen umsetzen. Die unterschiedliche Abschwächung des Röntgenstrahls beruht auf der unterschiedlichen Verteilung von Kontrastmitteln im Gewebe. Elektronen- und Kernspin-Tomographen sind Geräte, die die Spinänderungen von Elektronen bzw. Atomkernen in starken, gerichteten Magnetfeldern erfassen und in Bildinformationen umsetzen.

Unter einem Linker werden im Sinne der Erfindung solche Verbindungen verstanden, die als Kupplungsglied oder "Spacer" zwischen Protein und aktivem Agens eingesetzt werden können. Meist handelt es sich hierbei um bifunktionelle Verbindungen, die mit einer funktionellen Gruppe eine chemische Bindung zu dem aktiven Agens und mit der zweiten funktionellen Gruppe eine chemische Bindung zu dem Protein, welches vom Tumor spezifisch oder unspezifisch aufgenommen wird, eingehen. Sofern es sich einrichten läßt (beispielsweise durch Radiojodmarkierung) kann der Linker auch die Funktion des aktiven Agens übernehmen. Beispiele für Linker sind:

2,4-Dichlorpyrimidin:

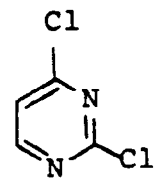

4,4'-Di-isothiocyano-2,2'-disulfonsäure stilbene (DIDS) :

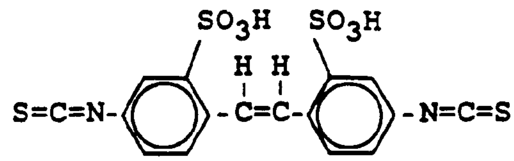

Cyanurchlorid (2,4,6-Trichlor-s-triazin) :

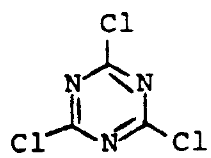

Ein Protein, das spezifisch oder unspezifisch vom Tumor aufgenommen wird, ist ein körpereigenes (autologes) Protein, das vom Abwehrsystem des Körpers nicht abgefangen wird und nur dem natürlichen Katabolismus unterliegt und den folgenden Bedingungen genügt:

a) es sollte eine möglichst lange biologische Halbwertzeit aufweisen, vorzugsweise von 2 bis 30 Tagen, insbesondere von 5 bis 10 Tagen;

b) es sollte oberhalb der glomerulären Ausschlußgrenze liegen, bevorzugt ein Molekulargewicht oberhalb von 50 kD besitzen, besonders bevorzugt oberhalb von 60 kD;

c) es sollte entweder:

1. eine Affinität zu tumorassoziierten Antigenen (spezifische Tumoranreicherung) oder
2. eine hohe Umsatzrate in Tumorgeweben (unspezifische Tumoraufnahme) aufweisen.

Bei einer möglichst langen biologischen Halbwertszeit bleibt das Protein ausreichend lange in der Zirkulation, so daß die Wahrscheinlichkeit der Anreicherung im Tumor (spezifisch oder unspezifisch) wesentlich höher ist.

Ein Molekulargewicht von oberhalb 50 kD ist vorteilhaft, weil:

1. Kleine Moleküle aufgrund der hohen Rückdiffusionskoeffizienten eine wesentlich geringere Verweildauer im Tumorgewebe aufweisen als Makromoleküle.
2. Proteine mit Molekulargewichten unter 50 kD von der Niere nicht zurückgehalten, sondern ausgeschieden werden.

Proteine mit einer hohen Affinität zu tumorassoziierten Antigenen sind bekannt, z. B. Antikörper oder Antikörperfragmente mono- oder polyclonaler Art.

Zu den Proteinen, die durch unspezifische Prozesse in Tumoren angereichert werden, gehören beispielsweise Serum-Albumin, Fibrinogen, Immunglobuline (IgG, IgM) sowie Lipoproteine.

Die erfindungsgemäßen Konjugate können beispielsweise zusammengesetzt werden aus einem oder mehreren Polyalkoholen, die chemisch an ein radioaktiv markiertes Agens (aktives Agens) gebunden sind und über einen Linker an ein autologes, spezifisch oder unspezifisch aufnehmbares Protein gekoppelt werden. Ein Beispiel hierfür ist das Konjugat der schematisierten Formel III:

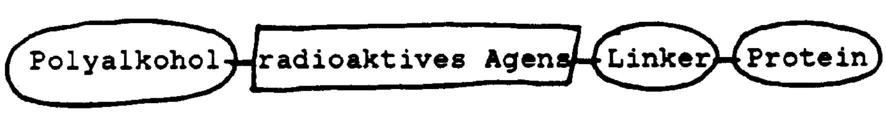


(III)

Das Konjugat eignet sich aufgrund seines integrierten radioaktiven Markers zur Tumorszintigraphie. Andere erfindungsgemäße Konjugate enthalten zwei unterschiedliche aktive Agenzien bzw. ein bifunktionelles Agens, wie das Konjugat der schematisierten Formel IV:

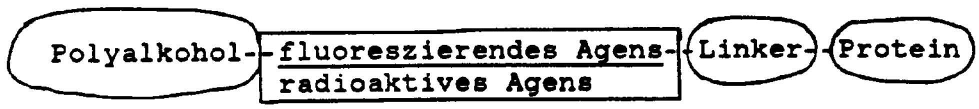

(IV)

Das Konjugat der Formel IV weist den Vorteil auf, daß unter Ausnutzung des radioaktiven Markers mittels szintigraphischer Methoden zunächst der Zeitpunkt der maximalen Anreicherung im Tumorgewebe bestimmt werden kann. Anschließend kann unter Ausnutzung der Fluoreszenzeigenschaften des selben Agenzes oder eines zweiten Agenzes mittels Fluoreszenzmikroskopie die Verteilung im Gewebe oder in Gewebeschnitten (z. B. aus Biopsieproben) exakt bestimmt werden.

Bei Verwendung von Konjugaten der Formel IVa kann man auf den radioaktiven Marker verzichten:

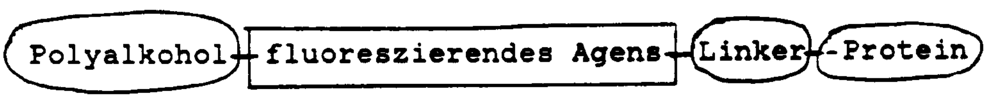

(IVa)

Speziell für diese Anwendung eignen sich beispielsweise Derivate von Porphyrinen, Phthalocyaninen und Naphthalocyaninen, die für die "Photodynamische Therapie" (PDT) mit Lasern von Bedeutung sind.

Konjugate der schematisierten Formel V:

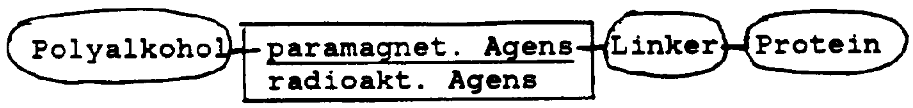

(V)

stellen erfindungsgemäße Konjugate dar, in denen das aktive Agens Atome mit ungerader Spinzahl ($^{13}C$, $^{15}N$, $^{19}F$, $^{31}P$ etc.) enthält, wobei das Agens als Kernspinmarker dient. Der Polyalkohol kann selbst das aktive Agens sein. Beide Arten von Kernspinmarkern können z. B. durch Einführung von $^{19}F$-haltigen Gruppen dargestellt werden. Die Konjugate der Formel V können so mit Hilfe der Kernspintomographie im Körper nichtinvasiv lokalisiert werden.

Durch den Tausch des signalgebenden aktiven Agenzes gegen ein therapeutisch aktives Agens erhält man beispielsweise erfindungsgemäße Konjugate der schematisierten Formel VI, die such zur Tumortherapie eignen:

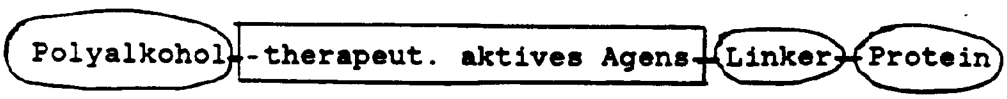

(VI)

Als therapeutische aktive Agenzien können beispielsweise photodynamisch aktive Substanzen wie Porphyrine, Phthalocyanine und Naphthalocyanine bzw. deren Derivate eingebaut werden. Nach Inkorporation im Tumorgewebe können diese Verbindungen durch Laserlicht von außen aktiviert werden, wobei hauptsächlich singuletter Sauerstoff entsteht. Dadurch werden gezielt zelluläre Makromoleküle im Tumorgewebe irreversibel oxidiert. Genauso gut lassen sich cytostatisch wirksame Substanzen (platinhaltige Verbindungen, Jod-Desoxycytidin, Cytosin-Arabinosid (Ara C), Anthracyclinderivate etc.) einbauen, die - im Tumorgewebe akkumuliert - gezielt ihre Wirkung entfalten. Es ist auf diese Weise möglich, cytostatisch wirksame Substanzen in höheren Konzentrationen ins Tumorgewebe zu transportieren und dort zu akkumulieren, ohne das Normalgewebe des Patienten zu belasten.

Um die Einzelverbindungen Polyalkohol, aktives Agens, Linker und Protein zu den erfindungsgemäßen Konjugaten Polyalkohol-aktives Agenz-Linker-Protein zusammenfügen zu können, sollten bevorzugt mindestens das oder die aktiven Agenzien und der oder die Linker jeweils zwei funktionelle Gruppen aufweisen, über die die chemischen Bindungen zwischen den einzelnen Molekülen hergestellt werden können. Solche funktionellen Gruppen sind beispielsweise Amino- oder Hydroxylgruppen, leicht substituierbare Halogenatome, wie sie beispielsweise im Cyanurchlorid vorhanden sind, Säurehalogenide, Carbonsäuren, Carbonsäureester wie N-Hydroxysuccinimidylester, Carbonsäureanhydride, Diazoniumgruppen oder Isothiocyanate. Sowohl die Polyalkohole als auch die Proteine weisen von sich aus mindestens eine reaktive funktionelle Gruppe wie eine Hydroxy-, Carboxy- oder Aminogruppe auf. Bei den aktiven Agenzien und den Linkern kann es gegebenenfalls

erforderlich sein, eine oder auch zwei solcher funktionellen Gruppen in das betreffende Molekül einzubauen. Dies geschieht zweckmäßigerweise nach literaturbekannten Verfahren.

Die erfindungsgemäßen Konjugate werden durch literaturbekannte chemische Verknüpfung der Einzelbausteine Polyalkohol, aktives Agenz, Linker und Protein hergestellt. Bevorzugt werden diese Einzelbausteine kovalent miteinander verknüpft. Hierbei ist es im allgemeinen vorteilhaft, wenn folgender Syntheseablauf eingehalten wird:

1. Kopplung des Polyalkohols an das aktive, mono- oder bifunktionelle Agens.
2. Sofern erforderlich, radioaktive Markierung, vorzugsweise mit Radiojod.
3. Umsetzung des gegebenenfalls radioaktiv markierten Reaktionsprodukts aus 2. mit dem Linker.
4. Umsetzung des Konjugats aus 3. mit dem jeweiligen Protein zum gewünschten Endprodukt. Sofern erforderlich, wird das so erhaltene erfindungsgemäße Konjugat noch gereinigt (beispielsweise über HPLC) und sterilisiert.

Als zweckmäßig hat es sich erwiesen, wenn man zunächst das aktive Agens oder die bereits aneinandergekoppelten aktiven Agenzien bevorzugt unter Zusatz von 0,8 bis 3, bevorzugt 1 bis 1,6 Mol (bezogen auf aktives Agens) Natriumcyanoborhydrid ($NaBH_3CN$) mit äquimolaren Mengen oder mit einem bis zu 2-fachen, bevorzugt bis zu 1,3-fachen Überschuß (bezogen auf aktives Agens) des Polyalkohols bei Temperaturen von 80 bis 120°C, bevorzugt 90 bis 110°C, 10 Min bis 10 Stunden, bevorzugt 20 Min bis 8 Stunden, umsetzt. Bevorzugt wird die Umsetzung in Gegenwart eines polaren Lösungsmittels wie Ethylenglycol, Glycerin oder Polyethylenglycol mit mindestens 2 Ethyleneinheiten und gegebenenfalls in Gegenwart eines Protonendonators, wie einer organischen oder anorganischen Säure, wie Essigsäure oder Salzsäure, durchgeführt. Die Reaktionstemperaturen liegen hierbei bei Raumtemperatur bis Siedetemperatur, bevorzugt bei 80 bis 120°C, besonders bevorzugt bei 90 bis 110°C und die Reaktionszeiten betragen 30 Min bis 4 Wochen, bevorzugt 10 Min bis 10 Stunden.

Sollen zwei oder mehr Polyalkoholmoleküle an das aktive Agens angekoppelt werden, so ist es zweckmäßig, zwei oder mehrere funktionelle Gruppen in das aktive Agens einzubauen, die mit dem Polyalkohol reagieren können. Es empfiehlt sich in diesem Fall, die eingesetzte molare Menge an Polyalkohol auf die im aktiven Agens befindlichen funktionellen Gruppen abzustimmen. In diesem Fall ist es auch zweckmäßig, die Menge an zugesetztem $NaBH_3CN$ zu erhöhen; vorzugsweise auf die äquimolare bis 1,5-fache Menge der im aktiven Agens befindlichen funktionellen Gruppen. Die Reaktionstemperaturen und Reaktionszeiten entsprechen denen der zuvor beschriebenen Umsetzung. Auch bei dieser Reaktion ist die Umsetzung in Gegenwart eines Lösungsmittels und gegebenenfalls eines Protonendonators, wie für die zuvor beschriebene Reaktion bevorzugt. Die Reaktionstemperaturen und Reaktionszeiten entsprechen ebenfalls denen der zuvor beschriebenen Umsetzung.

Werden radioaktiv markierte Verbindungen als aktive Agenzien verwendet, so kann - sofern die radioaktiv markierten Verbindungen als Ausgangssubstanzen nicht käuflich erhältlich sind - die Markierung mit dem Radioisotop vor, insbesondere jedoch nach der Umsetzung mit dem oder den Polyalkoholen erfolgen. In manchen Fällen ist es auch möglich, die radioaktive Markierung erst nach der Ankopplung des Linkers und sogar nach Ankopplung des Proteins durchzuführen. Es hat sich jedoch, beispielsweise bei Verwendung von radiojodmarkiertem Tyramin als aktivem Agens, als zweckmäßig erwiesen, die Radiojodmarkierung an dem Polyalkohol-Tyramin-Konjugat vorzunehmen. Die Radiojodmarkierung kann nach literaturbekannten Methoden erfolgen. Bevorzugt wird die Radiojodmarkierung mit einer Hypochloridlösung von genau definiertem pH-Wert und $Cl^+$-Gehalt durchgeführt. Als zweckmäßig hat sich eine bei Raumtemperatur gesättigte Chlorlösung erwiesen (pH = 7,4, $[Cl^+]$ = 0,1 μg/ml). Zur Markierung wird nun zu einer Vorlage aus zu markierendem Produkt und wäßriger alkalischer Radiojodid-Lösung (mit einer spez. Aktivität von 0,1 μg $^{131}J^-$/mCi$^{131}$J) so viel der oben beschriebenen Hypochloridlösung zugegeben, wie zur Oxidation der eingesetzten Menge an $^{131}J^-$ notwendig ist. Als zweckmäßig hat sich hierfür eine Konzentration von 0,2 bis 2 μg $Cl^+$/μg $^{131}J^-$ erwiesen. Die Reaktionstemperatur liegt bei Raumtemperatur und die Reaktionszeit beträgt 2 bis 30 Min. Die Ausbeuten liegen bei 90 bis 99 %. Das Produkt kann anschließend ohne weitere Reinigung weiterverarbeitet werden.

Soll der Polyalkohol die Doppelfunktion Polyalkohol + aktives Agens übernehmen, so empfiehlt es sich, die entsprechende Derivatisierung des Polyalkohols vor der Ankopplung an den Linker vorzunehmen. Hierzu wird der Polyalkohol nach literaturbekannten Verfahren beispielsweise in die $C^{19}F_3$-, $^{19}$F-Alkyl-, $^{19}$F-Phenyl- oder $^{19}$F-Derivate überführt.

Das erhaltene Polyalkohol-aktives Agens-Konjugat kann nun in einem weiteren Reaktionsschritt mit einem Linker umgesetzt werden. Diese Umsetzung erfolgt ebenfalls nach literaturbekannten Verfahren. Bevorzugt wird das Polyalkohol-aktives Agens-Konjugat mit Cyanurchlorid (2,4,6-Trichlor-s-triazin) umgesetzt.
Dazu wird das Polyalkohol-aktives Agens-Konjugat mit 0,8 bis 2, bevorzugt 1 bis 1,3 Moläquivalenten (bezogen auf das Polyalkohol-aktives Agens-Konjugat) Cyanurchlorid bei Temperaturen von 4 bis 30°C, bevorzugt 18 bis 22°C umgesetzt. Die Reaktionszeit beträgt 1 bis 5 Minuten, bevorzugt 2 bis 3 Minuten. Besonders bevorzugt erfolgt die Umsetzung in einem Lösungsmittel wie Phosphatpuffer (pH 7,4), Dimethylacetamid oder Mischungen derselben. Auch in diesem Fall gelten die oben angegebenen Reaktionstemperaturen und Reaktionszeiten. Wird Cyanurchlorid über die endständige Hydroxylgruppe des radiojodaktiven Tyramins gebunden, so hat dies weiterhin den Vorteil, daß das in ortho-Position zur OH-Gruppe des Tyramins stehende radioaktive Jod nach Etherbrückenbildung zum Triazinmolekül fester an den Benzolring des Tyramins gebunden wird, wodurch eine allzu leichte und bei Radiojod-Diagnostika gemäß Stand der Technik oft zu beobachtende Dejodierung im Organismus vermieden wird.

Das so erhaltene Polyalkohol-aktives Agens-Linker-Konjugat wird im letzten Reaktionsschritt mit dem Protein umgesetzt. Auch diese Umsetzung kann nach literaturbekannten Verfahren erfolgen. Bei der bevorzugten Verwendung von Cyanurchlorid als Linker erfolgt die Ankopplung des Proteins über die noch freie funktionelle Gruppe (ein Chloratom) des Cyanurchlorids unter Ausbildung einer Ether-, Thio- oder Aminobrücke, je nachdem, über welche Aminosäure des Proteins die Bindung erfolgt.

Zur Durchführung dieser Reaktion wird das Polyalkoholaktives Agens-Linker-Konjugat mit äquimolaren bis 2-fach molaren Mengen, bevorzugt äquimolaren Mengen des entsprechenden Proteins umgesetzt. Die Reaktionstemperaturen betragen hierbei 4 bis 30°C, bevorzugt 18 bis 22°C. Die Reaktionszeiten betragen 10 bis 60 Minuten, bevorzugt 30 bis 40 Minuten. Auch bei dieser Reaktion wird bevorzugt in einem Lösungsmittel wie Phosphatpuffer (pH 7,4 bis 8,5) mit oder ohne Zusatz von Dimethylacetamid gearbeitet. Auch hierfür gelten die oben angegebenen Reaktionstemperaturen und -zeiten.

Die erfindungsgemäßen Konjugate lassen sich auch durch beliebiges Vertauschen der oben beschriebenen Verfahrensschritte herstellen, solange sichergestellt ist, daß die Molekülabfolge Polyalkohol-aktives Agens-Linker-Protein entsteht.

Die Isolierung und Reinigung der erfindungsgemäßen Konjugate erfolgt, gegebenenfalls nach Aufkonzentrierung durch Zentrifugation in ®AMICON-C30-C100-Zellen, beispielsweise über HPLC. Die gegebenenfalls erforderliche Sterilisation erfolgt beispielsweise über 0,22 μm Sterilfilter.

Die zur Herstellung der erfindungsgemäßen Konjugate notwendigen Ausgangssubstanzen können, soweit sie nicht käuflich erhältlich sind, auf einfache Weise nach literaturbekannten Verfahren hergestellt werden.

Die erfindungsgemäßen Konjugate können nach Reinigung und Sterilisation entweder sofort appliziert oder zuvor mit einem geeigneten Lösungsmittel versetzt werden. Als Lösungsmittel kommen z. B. in Frage physiologische Kochsalzlösung oder Phosphatpuffer (pH 7,4; 287 mOsm), daneben auch Zuckerlösungen wie Glucose- oder Mannitlösungen, oder auch Mischungen aus den verschiedenen genannten Lösungsmitteln. Als zweckmäßig für den diagnostischen Einsatz haben sich solche injizierbare Zusammensetzungen erwiesen, die die erfindungsgemäßen Konjugate in Konzentrationen von 1 bis 10 mg/ml, bevorzugt ca. 4 mg/ml enthalten. Für den therapeutischen Einsatz empfehlen sich Konjugatkonzentrationen von 40 bis 50 mg/ml.

Bei den Konjugaten, die ein radioaktiv markiertes Label oder ein Radiopharmakon enthalten, empfiehlt es sich, diese erst kurz vor der Anwendung herzustellen, da sich somit leichter die therapeutisch erforderliche bzw. diagnostisch notwendige Strahlendosis einstellen läßt. Insbesondere wenn die erfindungsgemäßen Konjugate Radionuklide mit kurzen Halbwertszeiten enthalten, hat sich die Synthese kurz vor der Anwendung bewährt.

Die erfindungsgemäßen injizierbaren Zusammensetzungen können Patienten mit Verdacht auf Tumor in Mengen von 1 bis 20 ml, bevorzugt 2 bis 5 ml injiziert werden. 24 bis 72 Stunden, bevorzugt 48 bis 72 Stunden nach der Injektion können dann mittels einer auf das verwendete aktive Agenz abgestimmten externen Abtastvorrichtung der oder die Tumore lokalisiert werden, bzw. für den Fall, daß das aktive Agenz von einer externen Vorrichtung initialisiert werden muß (beispielsweise bei der therapeutischen Behandlung mit photoaktiven Substanzen), kann spätestens nach dieser Zeit die Initialisierung vorgenommen werden.

## Beispiele

### Beispiel 1

Herstellung von Tyramin-N-1′-Desoxysorbitol (TDS)

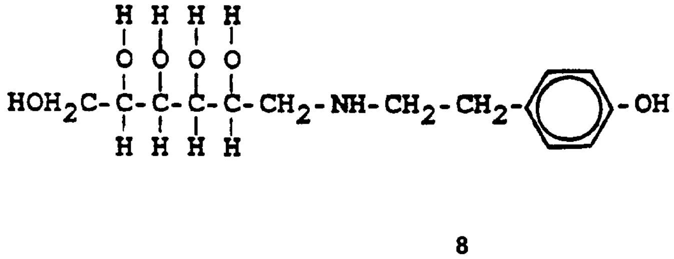

34,8 mg Tyramin x HCl (0,2 mMol) (Hersteller, Serva, Heidelberg) werden zusammen mit 50 mg D(+)Glucose-Monohydrat (etwa 0,25 mMol) (Hersteller: E. Merck, Darmstadt) in 1 ml Ethylenglykol unter vorsichtiger Erwärmung gelöst. Anschließend werden 20 mg Natriumcyanoborhydrid (etwa 0,3 mMol) (Hersteller: E. Merck, Darmstadt) zugegeben und die Reaktionsmischung 30 min im Glycerinbad auf 100°C erhitzt. Nach dem Erkalten der Lösung wird mit 9 ml $H_2O$ dest. verdünnt. Die so hergestellte Lösung kann direkt zur Analyse oder Trennung der Komponenten mittels HLPC eingesetzt werden.

HPLC-Bedingungen:

| | |
|---|---|
| Vorsäule: | C18-20 µm, 50 x 4 mm, (Fa. Latek, Heidelberg) |
| Säule: | Nucleosil 10 SA, 250 x 4 mm, (Fa. Latek, Heidelberg) |
| Eluent: | 0,1 M $NH_4$-Acetat, pH 6,0, 90 %<br>Acetonitril 10 % |
| Fluß: | 1 ml/min |
| UV-Detektor: | 280 nm |

Retentionszeiten:

1) unbek. Komponente: 6,55 min
2) Tyramin-N-1'-Desoxysorbitol: 7,80 min
3) Tyramin: 8,90 min

Laut HPLC beträgt die Ausbeute etwa 75 - 80 %.

**Beispiel 2**

Radioaktive Markierung von Tyramin-N-1'-Desoxysorbitol (TDS) mit Radiojod ($^{131}J$)

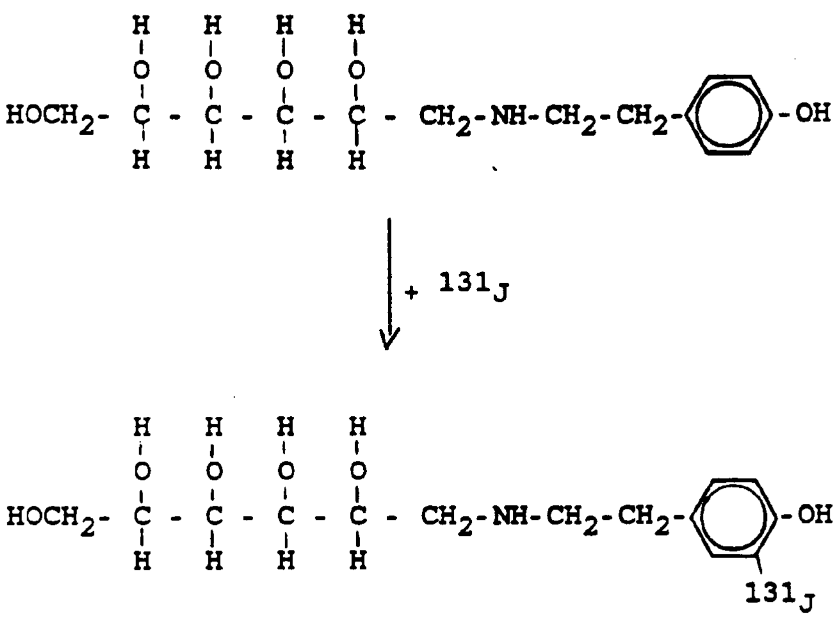


25 µg TDS (0,08 µmol), gelöst in 25 µl 0,13 M Phosphatpuffer (pH 7,4), werden bei Raumtemperatur mit einer wäßrigen, alkalischen Lösung (pH 8 - 11) von Radiojod (bis 10 mCi$^{131}$I entsprechend einer Menge von etwa 1 µg Jod) versetzt. Durch Zugabe von 5 bis 10 µl einer Natriumhypochloridlösung (0,1 µg $Cl^+$/µl Phosphatpuffer pH 7,4) pro mCi Radiojod ($^{131}J$) wird das Radiojod-Jodid in das reaktive Jodonium Ion ($J^+$) überführt, das nun seinerseits durch elektrophile Substitution ein zur Hydroxylgruppe ortho-ständiges Proton am Phenylring ersetzt.
Markierungsausbeute: 95 bis 98 %
Dünnschichtchromatographie:

| Laufmittel: | Aceton, | Butanol-1, | 25 % Ammoniak, | dest. $H_2O$ |
|---|---|---|---|---|
| | 60 | 25 | 10 | 5 (v/v) |

Platten: Kieselgel 60 (5 x 20 cm) ohne Fluoreszenzindikator (E. Merck, Darmstadt, FRG)
Laufstrecke: 10 cm

| Rf.: | $^{131}$I-TDS | 0,4 bis 0,45 |
|---|---|---|
| | $^{131}$I-Iodid | 0,94 |

**Beispiel 3**

Umsetzung von $^{131}$I-TDS aus Beispiel 2 mit Cyanurchlorid:

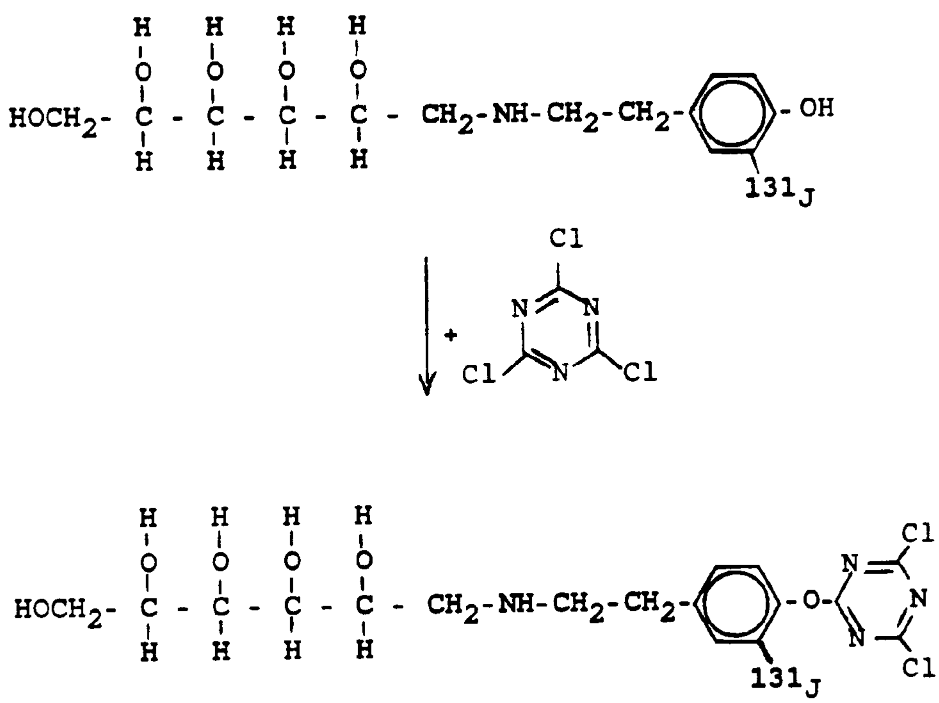

Für die Umsetzung von $^{131}$I-TDS mit Cyanurchlorid stellt man zunächst eine Lösung von Cyanurchlorid in wasserfreiem 1,4-Dioxan (10 mg/ml) her.

Zu der Lösung von $^{131}$I-TDS gibt man (bezogen auf 25 µg TDS) das 1,3-fache der theoretisch erforderlichen Menge an Cyanurchlorid (20 µg Cyanurchlorid, entsprechend 2 µl der zuvor gefertigten Dioxanlösung). 2 bis 3 Minuten nach erfolgter Cyanurchloridzugabe ist die Umsetzung bei Raumtemperatur beendet.

**Beispiel 4.1**

Umsetzung von $^{131}$I-TDS-di-chloro-triazin aus Beispiel 3 mit Rattenserumalbumin

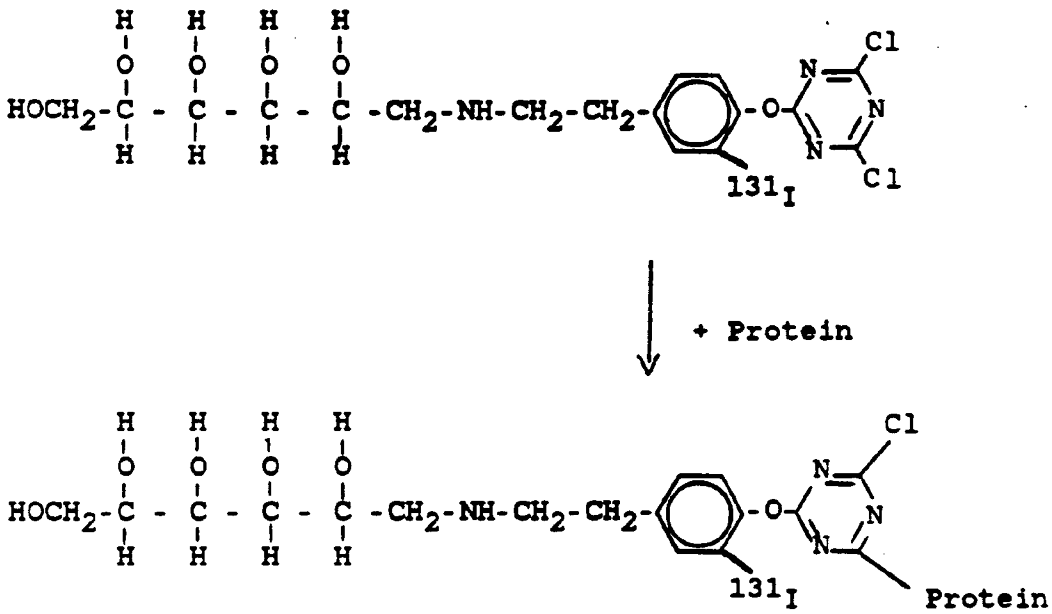


Zur Kopplung der aus Beispiel 3 resultierenden Verbindung $^{131}$I-TDS-di-chloro-triazin mit Rattenserumalbumin (ca. 70 kD) fügt man die äquimolare Menge des Proteins, gelöst in Phosphatpuffer (25 mg/ml, pH 8,3), hinzu. Die auf 25 µg TDS bezogene Menge Rattenserumalbumin beträgt 5,8 mg. Nach einer Reaktionszeit von 30 bis 40 Minuten bei Raumtemperatur beträgt die Kopplungsausbeute 80 bis 85 %.

**Beispiel 4.2**

Analog zu Beispiel 4.1 wird $^{131}$J-TDS-di-chloro-triazin (aus Beispiel 3) mit Ratten-IgG (ca. 150 kD) umgesetzt. Die erforderliche Menge an Ratten IgG beträgt 12,5 mg. Die Kopplungsausbeute beträgt 80 bis 85 %.

**Beispiel 4.3**

Analog zu Beispiel 4.1 wird $^{131}$J-TDS-di-chloro-triazin (aus Beispiel 3) mit Fibrinogen (ca. 350 kD) umgesetzt. Die erforderliche Menge an Fibrinogen beträgt 30 mg. Die Kopplungsausbeute beträgt 80 bis 85 %.

**Beispiel 5**

Herstellung von 4-Amino-1,8-Naphthalsäuretyrimid (ANT)

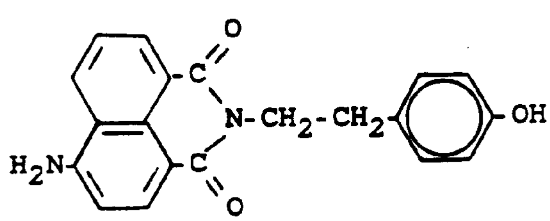

426 mg 4-Amino-1,8-naphthalinsäureanhydrid (2 mmol) (Hersteller: Aldrich-Chemie, Heidenheim) werden zusammen mit 1,37 g Tyramin (10 mmol) (Hersteller: Aldrich-Chemie, Heidenheim) in 30 ml Dimethylformamid (DMF) gelöst und 30 min im Glycerinbad auf 140°C erhitzt. Das DMF wird im Rotationsverdampfer im Ölpumpenvakuum abgezogen und der Rückstand mit 100 ml 6 N HCl 2,5 h am Rückfluß gekocht. Nach dem Erkalten der salzsauren Lösung wird der braungelbe Bodenkörper abfiltriert, in einer heißen Mischung aus 50 ml Methanol und 50 ml Ethanol gelöst und durch Zugabe von 300 ml 6 N HCl wieder ausgefällt. Die Fällung wird

vervollständigt, wenn die Lösung für 2 Stunden bei +4°C aufbewahrt wird. Der Bodenkörper wird abfiltriert und im Exsiccator getrocknet.
Ausbeute: 498 mg (etwa 75 % d. Th.).

Analytik:

1. Dünnschichtchromatographie:

| Laufmittel: | Aceton, | Butanol-1, | 25 % Ammoniak, | dest. $H_2O$ |
|---|---|---|---|---|
| | 60 | 25 | 10 | 5 (v/v) |

Platten: Kieselgel 60 (5 x 20 cm) ohne Fluoreszenzindikator (E. Merck, Darmstadt, FRG)
Laufstrecke: 10 cm
Rf.: 4-Amino-1,8-naphthalinsäuretyrimid: 0,88 - 0,92 (die Substanz zeigt eine intensive gelbgrüne Fluoreszenz und besitzt ein Absorptionsmaximum bei 445 nm).

2. HPCL:

| Bedingungen | |
|---|---|
| Probevolumen: | 100 µl |
| Vorsäule : | 50 x 4 mm; C18-30µ |
| Säule : | 250 x 10 mm; C18-5µ GO |
| Eluent : | A: 0,1 % Ameisensäure in Wasser 40 % |
| | B: Methanol 60 % |
| Gradient : | 60 - 100 % Methanol in 12 Min.; Exponent 2 |
| Delay-time : | 2 Min. |
| Fluß : | 4 ml/Min. |
| $\lambda_{uv}$ : | 445 nm |
| Retentionszeit : | 11,4 Min. |

**Beispiel 6**

Herstellung von 4-Amino-1,8-naphthalinsäuretyrimid-N-1′-desoxysorbitol (ANT-N-1′-Desoxysorbitol)

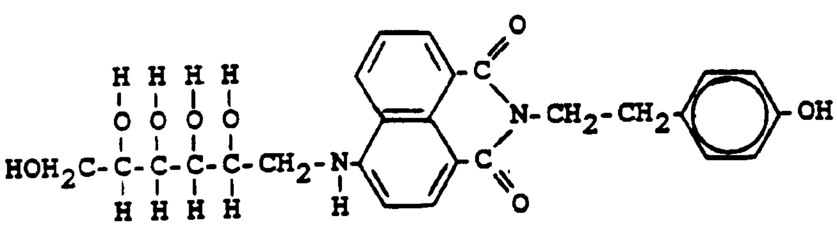

10 mg ANT (30 µMol) aus Beispiel 5 werden zusammen mit 200 mg Glucose-Monohydrat (etwa 1 mMol) (Hersteller: E. Merck, Darmstadt) und 68 mg Natriumcyanoborhydrid (etwa 1,1 mMol) (Hersteller: E. Merck, Darmstadt) in 1 ml Ethylenglykol und 0,3 ml 25 % Essigsäure gelöst und im Glycerinbad 5 h auf 100°C erhitzt. Nach dem Erkalten der Reaktionslösung wurden ANT und ANT-N-1′-Desoxysorbitol durch eine HPLC-Vorreinigung aus dem Reaktionsgemisch abgetrennt.
Säule: Nucleosil C18-20 µm, 100 x 10 mm
Eluent: 0,1 % Ameisensäure in Wasser
Fluß: 3 ml/min
Auftragsvol.: 500 µl

Nach 5 min wurde das Lösungsmittel gewechselt und ANT und ANT-N-1′-Desoxysorbitol mit 100 % Methanol von der Säule eluiert.

Die Trennung von ANT und ANT-N-1′-Desoxysorbitol erfolgte unter denselben HPLC-Bedingungen, die zur ANT-Identifikation dienten.

| Retentionszeit: | |
|---|---|
| ANT-N-1′-Desoxysorbitol: | 8,55 min |
| ANT: | 11,40 min |

Nach Abtrennung des Laufmittels resultierte eine Ausbeute von 3 mg (etwa 20 % d. Th.).

**Beispiel 7**

Radioaktive Markierung von ANT-N-1'-DS mit Radiojod

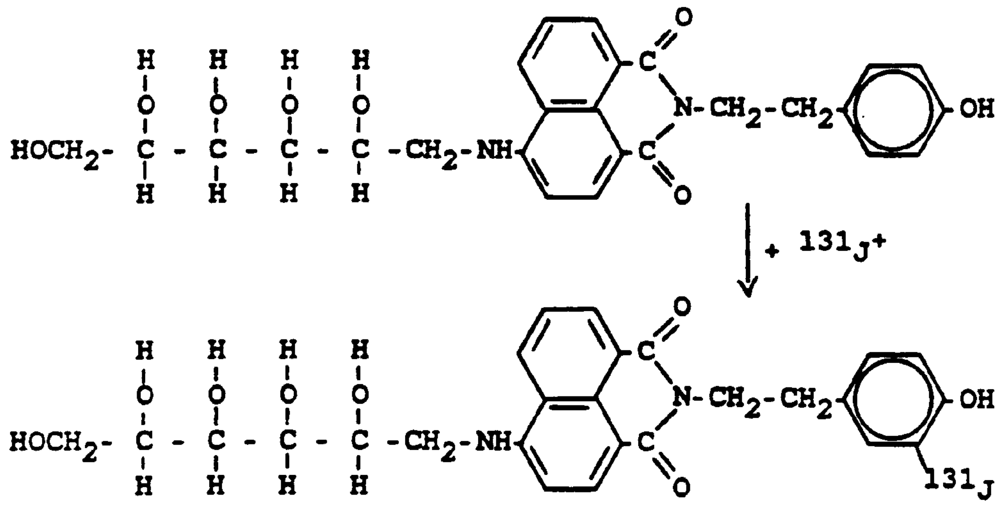


50 µg ANT-N-1'-DS (etwa 0,1 µmol) aus Beispiel 6, gelöst in 100 µl einer Mischung aus 0,13 M Phosphatpuffer (pH 7,4) und Dimethylacetamid (70 : 30) werden mit einer wäßrigen, alkalischen (pH 8 bis 11) Lösung von Radiojod (bis 10 mCi $^{131}I$ entsprechend einer Menge von etwa 1 µg Jod) versetzt. Durch Zugabe von 5 bis 10 µl einer Natriumhypochloridlösung (0,1 µg $Cl^+$/µl Phosphatpuffer pH 7,4) pro mCi Radiojod ($^{131}I$) wird das Radiojod-Jodid in das reakive Jodonium Ion ($J^+$) überführt, das nun seinerseits durch elektrophile Substitution ein zur Hydroxylgruppe ortho-ständiges Proton am Phenylring ersetzt.
Markierungsausbeute: 95 - 98 %.
Analytik: Dünnschichtchromatographie

| Laufmittel: | Aceton, | Butanol-1, | 25 % Ammoniak, | dest. $H_2O$ |
|---|---|---|---|---|
| | 60 | 25 | 10 | 5 (v/v) |

Platten: Kieselgel 60 (5 x 20 cm) ohne Fluoreszenzindikator (E. Merck, Darmstadt, FRG)
Laufstrecke: 10 cm

Rf.: $^{131}I$-ANT-N-1'-DS: 0,44 - 0,48
$^{131}I$-Iodid : 0,94

**Beispiel 8**

Umsetzung von $^{131}$I-ANT-N-1'-DS mit Cyanurchlorid:

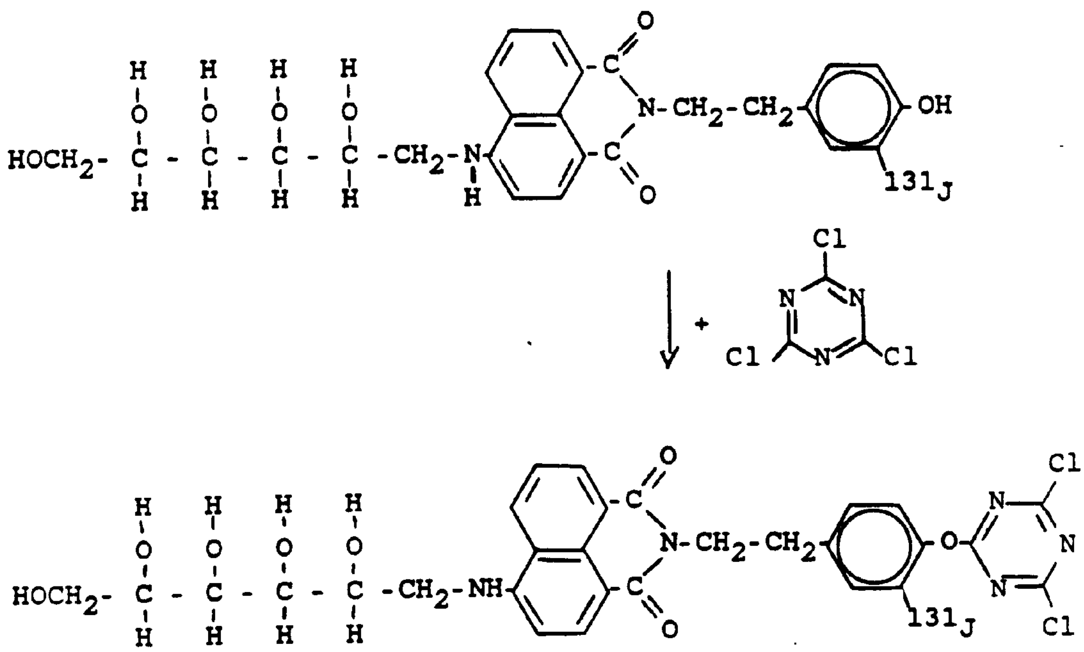

Für die Umsetzung von $^{131}$I-ANT-N-1'-DS aus Beispiel 7 mit Cyanurchlorid stellt man zunächst eine Lösung von Cyanurchlorid in wasserfreiem 1,4-Dioxan (10 mg/ml) her. Für eine äquimolare Umsetzung gibt man zu der Lösung von $^{131}$I-ANT-N-1'-DS (bezogen auf 50 µg ANT-N-1'-DS) das 1,3-fache der theoretisch erforderlichen Menge an Cyanurchlorid (24,5 µg Cyanurchlorid entsprechend 2,5 µl der zuvor gefertigten Dioxanlösung). 2 bis 3 min nach erfolgter Cyanurchloridzugabe ist die Umsetzung bei Raumtemperatur beendet.

**Beispiel 9.1**

Umsetzung von $^{131}$I-ANT-N-1'-DS-di-chloro-triazin mit Rattenserumalbumin

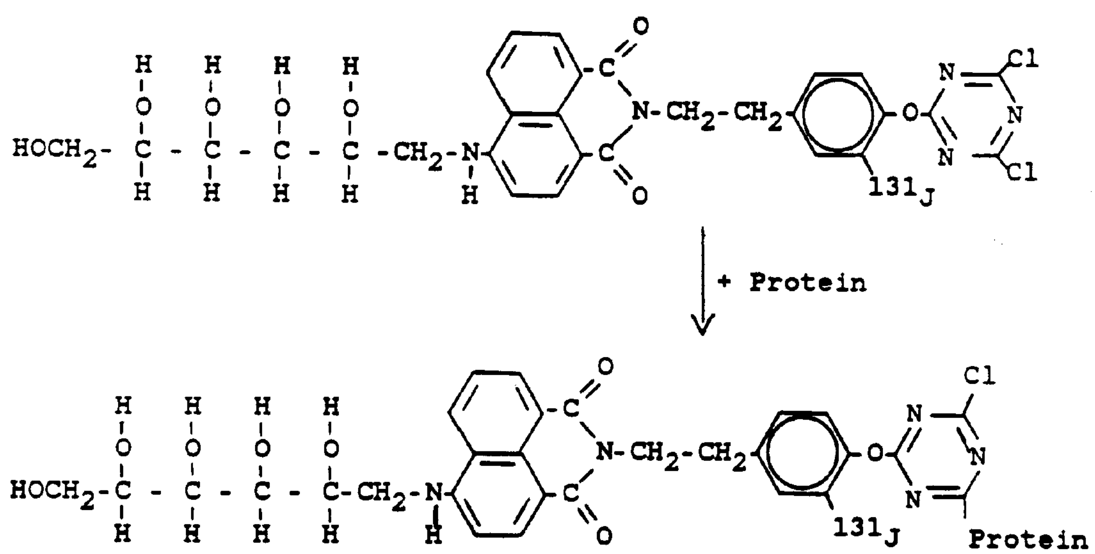

Zur Kopplung der aus Beispiel 8 resultierenden Verbindung $^{131}$I-ANT-N-1′-DS-di-chloro-triazin mit Rattenserumalbumin (ca. 70 kD) fügt man die äquimolare Menge des Proteins, gelöst in Phosphatpuffer (25 mg/ml, pH 8,3), hinzu. Die auf 50 μg ANT-N-1′-DS bezogene Menge an Rattenserumalbumin beträgt 7 mg. Nach einer Reaktionszeit von 30 bis 40 Minuten bei Raumtemperatur beträgt die Kopplungs- und Proteinmarkierungsausbeute etwa 80 %.

Die Reinigung des markierten Proteins erfolgt über HPLC:
Pumpe: P 400 (Fa. Latek, Heidelberg, FRG)
Eluent: 0,2 M Natriumphosphat pH 7,4
Fluß: 1 ml/min
Vorsäule: 50 x 4 mm, Zorbax Diol
Säule I: Zorbax GF 450 (Fa. DuPont, Dreieich, FRG)
Säule II: Zorbax GF 250
UV-Detektor: Single Path Monitor UV-1 (Fa. Pharmacia, Freiburg, FRG)
γ-Detektor: NaI 1,5″ x 2″ mit Berthold Elektronik
Rekorder: Shimadzu, Chromatopac C-R1A (Fa. Latek)
Retentionszeit: 19,8 min

**Beispiel 9.2**

Analog zu Beispiel 9.1 wird $^{131}$J-ANT-N-1′-DS-di-chloro-triazin (aus Beispiel 8) mit Ratten-IgG (ca. 150 kD) umgesetzt. Die erforderliche Menge an Ratten-IgG beträgt 15 mg. Die Proteinmarkierungsausbeute liegt bei 80 %. Die Reinigung erfolgt über HPLC, wie in Beispiel 9.1 beschrieben.
Retentionszeit: 18,2 Min.

**Beispiel 9.3**

Analog zu Beispiel 9.1 wird $^{131}$J-ANT-N-1′-DS-di-chloro-triazin (aus Beispiel 8) mit Fibrinogen (ca. 350 kD) umgesetzt. Die erforderliche Menge an Fibrinogen beträgt 35 - 37 mg. Die Proteinmarkierungsausbeute liegt bei 80 %. Die Reinigung erfolgt über HPLC, wie in Beispiel 9.1 beschrieben.
Retentionszeit: 15,0 Min.

**Pharmakologische Beispiele**

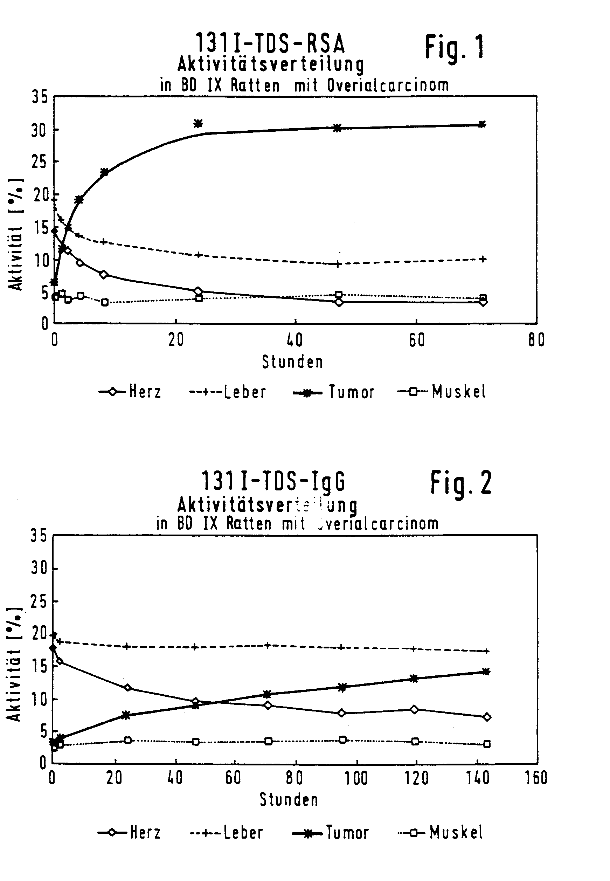

Zum Nachweis der hohen Tumoranreicherungsrate der erfindungsgemäßen Konjugate wurden diese Versuchstieren, denen zuvor ein Tumor implantiert worden war, injiziert. Die prozentuale Aktivitätsverteilung (bezogen auf applizierte Aktivität) in Herz, Leber, Tumor und Muskel von verschiedenen erfindungsgemäßen Konjugaten, welche als aktive Agenzien ein radioaktives Label enthielten, wurde in Abhängigkeit der Zeit gemessen. Die Aktivitätsverläufe sind den Fig. 1 und 2 zu entnehmen.

**Tierversuch mit $^{131}$I-TDS-RSA (Tyramin-desoxy-sorbitol, gekoppelt an Ratten Serum-Albumin, Substanz aus Bsp. 4.1**

Aufnahmegerät: γ-Kamera (Searle, Pho-Gamma IV, FLOV)
Datenerfassungsgerät: Gaede, medworker N 4)
Datenerfassungsmode: Frame mode
Matrix: 128 x 128
Tiermodell: Ratte, BD IX, weiblich
Alter: 8 Wochen
Gewicht: 200 - 220 g
Anzahl der Tiere: 4
Tumor: Ovarial-Karzinom (0 - 342, Zeller, DKFZ)
Implantationsart: intramuskulär
Implantierte Zellzahl: 5 x $10^6$
Implantationsort: hintere Extremität, links
Versuchsbeginn: 10 Tage nach Tumortransplantation
Tumordurchmesser bei Versuchsbeginn: 15 - 18 mm
Versuchsdauer: 72 Stunden
Applikationsart des $^{131}$I-TDS-RSA: intravenös

Applikationsort: laterale Schwanzvene
Applizierte Aktivitätsmenge: 300 µCi (11,1 mBq) $^{131}I$
Applikationsvolumen: 0,3 ml
Lösungsmittel: Phosphatpuffer (PPB), 0,13 M, pH 7,4
Spez. Aktivität: 1 mCi (37 MBq)/5 µg TDS x 1 mg RSA x 1 ml PPB
Applizierte Proteinmenge: 0,3 mg (entsprechend 0,3 mCi)
Aufnahmezeitpunkte: 10 min, 1, 2, 4, 18, 48, 72 h p.i.
Aufnahmezeit: 5 min.
Tumordurchmesser am Ende des Versuchs: 20 - 22 mm

Die Aktivitätsverteilung ist in Fig. 1 dargestellt.

**Tierversuch mit $^{131}I$-TDS-IgG (Tyramin-desoxy-sorbitol, gekoppelt an Ratten IgG, Substanz aus Beispiel 4.2**

Aufnahmegerät: γ-Kamera (Searle, Pho-Gamma IV, FLOV)
Datenerfassungsgerät: Gaede, medworker N 4)
Datenerfassungsmode: Frame mode
Matrix: 128 x 128
Tiermodell: Ratte, BD IX, weiblich
Alter: 8 Wochen
Gewicht: 200 - 220 g
Anzahl der Tiere: 4
Tumor: Ovarial-Karzinom (0 - 342, Zeller, DKFZ)
Implantationsart: intramuskulär
Implantierte Zellzahl: 5 x $10^6$
Implantationsort: hintere Extremität, links
Versuchsbeginn: 10 Tage nach Tumortransplantation
Tumordurchmesser bei Versuchsbeginn: 10 - 12 mm
Versuchsdauer: 144 Stunden
Applikationsart des $^{131}I$-TDS-IgG: intravenös
Applikationsort: laterale Schwanzvene
Applizierte Aktivitätsmenge: 125 µCi (11,1 MBq) $^{131}I$
Applikationsvolumen: 0,3 ml
Lösungsmittel: Phosphatpuffer (PPB), 0,13 M, pH 7,4
Spez. Aktivität: 1 mCi (37 MBq)/2,5 µg TDS x 1 mg IgG x 1 ml PPB
Applizierte Proteinmenge: 0,125 mg (entsprechend 0,125 mCi)
Aufnahmezeitpunkte: 10 min, 1, 2, 4, 18, 48, 72, 96, 120, 144 h
Aufnahmezeit: 5 min.
Tumordurchmesser am Ende des Versuchs: etwa 20 mm

Die Aktivitätsverteilung ist in Fig. 2 dargestellt.

## Patentansprüche

### Patentansprüche für folgende Vertragsstaaten : AT, BE, CH, DE, DK, FR, GB, IT, LI, LU, NL, SE

**1.** Konjugat, bestehend aus
a) mindestens einem Polyalkohol oder einem derivatisierten Polyalkohol
b) mindestens einem aktiven Agens,
c) mindestens einem Linker und
d) einem Protein, wobei

der oder die Polyalkohole oder der oder die derivatisierten Polyalkohole vom Abwehrsystem eines Organismus nicht als körperfremd erkannte Polyalkohole oder derivatisierte Polyalkohole sind und das Protein ein vom Tumor spezifisch oder unspezifisch aufnehmbares, vom Abwehrsystem eines Organismus nicht als körperfremd erkanntes Protein ist, dadurch gekennzeichnet, daß der oder die Polyalkohole oder der oder die derivatisierten Polyalkohole eine Verbindung der Formel I sind,

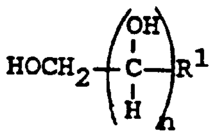 (I)

worin
$R^1$ $CH_2OH$, CHO oder $CH_2NH_2$ bedeutet und n 1 - 10 ist,
und worin eine

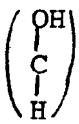 -Gruppe

durch CO ersetzt sein kann und worin keine, eine oder mehrere OH-Gruppen durch $NH_2$, $^{19}F$, $C^{19}F_3$, mono- oder poly-$^{19}F$-substituiertes $C_1$-$C_4$-Alkyl oder mono-, di-, tri-, tetra- oder penta-$^{19}F$-substituiertes Phenyl ersetzt sein können, oder der Polyalkohol Glucose, fructose, Maltose, Saccharose oder Sorbit ist, oder das Polyalkoholderivat Glucose, fructose, Maltose, Saccharose oder Sorbit ist, wobei mindestens eine Oh-Gruppe in diesen Verbindungen durch $^{19}F$, $C^{19}F_3$, mono- oder poly-$^{19}F$-substituiertes $C_1$-$C_4$Alkyl oder mono-, di-, tri-, tetra- oder penta-$^{19}F$-substituiertes Phenyl ersetzt ist.

2. Konjugat nach Anspruch 1, dadurch gekennzeichnet, daß der oder die derivatisierten, vom Abwehrsystem eines Organismus nicht als körperfremd erkannten Polyakohole oder der oder die Linker die Funktion des oder der aktiven Agenzien übernehmen.

3. Konjugat nach Anspruch 1 oder 2, dadurch gekennzeichnet, daß die Komponenten a), b), c) und d) miteinander kovalent verbunden sind.

4. Konjugat nach einem oder mehreren der Ansprüche 1 bis 3, dadurch gekennzeichnet, daß die Komponente a) aus einem vom Abwehrsystem eines Organismus nicht als körperfremd erkannten Polyakohol oder derivatisierten Polyalkohol besteht.

5. Konjugat nach einem oder mehreren der Ansprüche 1 bis 4, dadurch gekennzeichnet, daß die Komponente b) aus einem oder zwei aktiven Agenzien besteht.

6. Konjugat nach einem oder mehreren der Ansprüche 1 bis 5, dadurch gekennzeichnet, daß die Komponente c) aus einem Linker besteht.

7. Konjugat nach einem oder mehreren der Ansprüche 1 bis 6, dadurch gekennzeichnet, daß der oder die autologen, vom Abwehrsystem eines Organismus nicht als körperfremd erkannten Polyalkohole ausgewählt werden aus der Gruppe, bestehend aus
Glucose, Fructose, Maltose, Saccharose, Sorbit und Glycerinaldehyd.

8. Konjugat nach einem oder mehreren der Ansprüche 1 bis 7, dadurch gekennzeichnet, daß das oder die aktiven Agenzien ausgewählt werden aus
radioaktiv markierten Verbindungen, einem Fluoreszenzmarker, einem NMR-Marker, einem ESR-Marker, einer photoaktiven Verbindung, einer $^{10}B$-haltigen Verbindung, einem Zytostatikum.

9. Konjugat nach einem oder mehreren der Ansprüche 1 bis 8, dadurch gekennzeichnet, daß das oder die aktiven Agenzien ausgewählt werden aus Verbindungen der Formel II

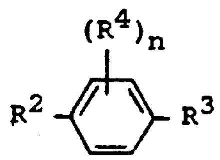

(II)

worin
$R^2$ $NH_2$, $C_1$-$C_4$-Alkylamin oder CHO bedeutet und
$R^3$ $NO_2$, $NH_2$, OH oder NCS bedeutet und
$R^4$ $^{131}J$, $^{123}J$, $^{19}F$, $C^{19}F_3$, OH, H bedeutet und
n 1 bis 4 ist.

**10.** Konjugat nach einem oder mehreren der Ansprüche 1 bis 9, dadurch gekennzeichnet, daß der oder die Linker ausgewählt werden aus
2,4-Dichloropyrimidin, 4,4′-Di-isothiocyano-2,2′-disulfonsäurestilben, Tyramin, Benzaldehyd und Cyanurchlorid.

**11.** Konjugat nach einem oder mehreren der Ansprüche 1 bis 10, dadurch gekennzeichnet, daß das vom Abwehrsystem eines Organismus nicht als körperfremd erkannte, spezifisch oder unspezifisch aufnehmbare Protein ausgewählt wird aus
autologem Serumalbumin, autologen polyklonalen oder monoklonalen Antikörpern oder Antikörperfragmenten und autologen Imunglobulinen.

**12.** Verfahren zur Herstellung des Konjugats nach Anspruch 1, dadurch gekennzeichnet, daß man zwischen den Komponenten a) und b) und c) und d) chemische Bindungen herstellt.

**13.** Verfahren nach Anspruch 12, dadurch gekennzeichnet, daß die chemischen Bindungen kovalent sind.

**14.** Tumordiagnostikum, enthaltend mindestens ein Konjugat nach einem oder mehreren der Ansprüche 1 bis 11.

**15.** Tumortherapeutikum, enthaltend mindestens ein Konjugat nach einem oder mehreren der Ansprüche 1 bis 11.

**16.** Verwendung mindestens eines Konjugats nach einem oder mehreren der Ansprüche 1 bis 11 zur Herstellung eines Tumordiagnostikums.

**17.** Verwendung mindestens eines Konjugats nach einem oder mehreren der Ansprüche 1 bis 11 zur Herstellung eines Tumortherapeutikums.

**18.** Verfahren zur Herstellung eines Tumortherapeutikums, dadurch gekennzeichnet, daß man mindestens ein Konjugat nach einem oder mehreren der Ansprüche 1 bis 11 mit einem physiologisch verträglichen Injektionsvehikel mischt.

**19.** Verfahren zur Herstellung eines Tumordiagnostikums, dadurch gekennzeichnet, daß man mindestens ein Konjugat nach einem oder mehreren der Ansprüche 1 bis 11 mit einem physiologisch verträglichen Injektionsvehikel mischt.

**Patentansprüche für folgende Verstragsstaaten : ES, GR**

**1.** Verfahren zur Herstellung eines Konjugats, bestehend aus
a) mindestens einem Polyalkohol oder einem derivatisierten Polyalkohol
b) mindestens einem aktiven Agens,
c) mindestens einem Linker und
d) einem Protein, wobei
der oder die Polyalkohole oder der oder die derivatisierten Polyalkohole vom Abwehrsystem eines Organismus nicht als körperfremd erkannte Polyalkohole oder derivatisierte Polyalkohole sind und das Protein ein vom Tumor spezifisch oder unspezifisch aufnehmbares, vom Abwehrsystem eines Organismus nicht als körperfremd erkanntes Protein ist, und bei dem man zwischen den Komponenten a), b), c)

und d) chemische Bindungen herstellt, dadurch gekennzeichnet, daß der oder die Polyalkohole oder der oder die derivatisierten Polyalkohole eine Verbindung der Formel I sind,

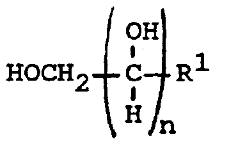

(I)

worin
$R^1$ $CH_2OH$, CHO oder $CE_2NH_2$ bedeutet und
n 1 - 10 ist,
und worin eine

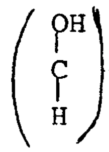-Gruppe

durch CO ersetzt sein kann und worin keine, eine oder mehrere OH-Gruppen durch $NH_2$, $^{9}F$, $C^{19}F_3$, mono- oder poly-$^{19}F$-substituiertes $C_1$-$C_4$-Alkyl oder mono-, di-, tri-, tetra- oder penta-$^{19}F$-substituiertes Phenyl ersetzt sein können, oder der Polyalkohol Glucose, fructose, Maltose, Saccharose oder Sorbit ist, oder das Polyalkoholderivat Glucose, fructose, Maltose, Saccharose oder Sorbit ist, wobei mindestens eine OH-Gruppe in diesen Verbindungen durch $^{19}F$, $C^{19}F_3$, mono- oder poly-$^{19}F$-substituiertes $C_1$-$C_4$Alkyl oder mono-, di-, tri-, tetra- oder penta-$^{19}F$-substituiertes Phenyl ersetzt ist.

2. Verfahren nach Anspruch 1, dadurch gekennzeichnet, daß der oder die derivatisierten, vom Abwehrsystem eines Organismus nicht als körperfremd erkannten Polyalkohole oder der oder die Linker die Funktion des oder der aktiven Agenzien übernehmen.

3. Verfahren nach Anspruch 1 oder 2, dadurch gekennzeichnet, daß die Komponenten a), b), c) und d) miteinander kovalent verbunden sind.

4. Verfahren nach einem oder mehreren der Ansprüche 1 bis 3, dadurch gekennzeichnet, daß die Komponente a) aus einem vom Abwehrsystem eines Organismus nicht als körperfremd erkannten Polyalkohol oder derivatisierten Polyalkohol besteht.

5. Verfahren nach einem oder mehreren der Ansprüche 1 bis 4, dadurch gekennzeichnet, daß die Komponente b) aus einem oder zwei aktiven Agenzien besteht.

6. Verfahren nach einem oder mehreren der Ansprüche 1 bis 5, dadurch gekennzeichnet, daß die Komponente c) aus einem Linker besteht.

7. Verfahren nach einem oder mehreren der Ansprüche 1 bis 6, dadurch gekennzeichnet, daß der oder die autologen, vom Abwehrsystem eines Organismus nicht als körperfremd erkannten Polyalkohole ausgewählt werden aus der Gruppe, bestehend aus
Glucose, Fructose, Maltose, Saccharose, Sorbit und Glycerinaldehyd.

8. Verfahren nach einem oder mehreren der Ansprüche 1 bis 7, dadurch gekennzeichnet, daß das oder die aktiven Agenzien ausgewählt werden aus
radioaktiv markierten Verbindungen, einem Fluoreszenzmarker, einem NMR-Marker, einem ESR-Marker, einer photoaktiven Verbindung, einer $^{10}B$-haltigen Verbindung, einem Zytostatikum.

9. Verfahren nach einem oder mehreren der Ansprüche 1 bis 8, dadurch gekennzeichnet, daß das oder die

aktiven Agenzien ausgewählt werden aus Verbindungen der Formel II

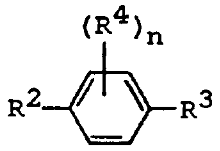

(II)

worin
$R^2$ $NH_2$, $C_1$-$C_4$-Alkylamin oder CHO bedeutet und
$R^3$ $NO_2$, $NH_2$, OH oder NCS bedeutet und
$R^4$ $^{131}J$, $^{123}J$, $^{19}F$, $C^{19}F3$, OH, H bedeutet und
n 1 bis 4 ist.

**10.** Verfahren nach einem oder mehreren der Ansprüche 1 bis 9 , dadurch gekennzeichnet, daß der oder die Linker ausgewählt werden aus
2,4-Dichloropyrimidin, 4,4'-Di-isothiocyano-2,2'-disulfonsäurestilben, Tyramin, Benzaldehyd und Cyanurchlorid.

**11.** Verfahren nach einem oder mehreren der Ansprüche 1 bis 10 dadurch gekennzeichnet, daß das vom Abwehrsystem eines Organismus nicht als körperfremd erkannte, spezifisch oder unspezifisch aufnehmbare Protein ausgewählt wird aus
autologem Serumalbumin, autologen polyklonalen oder monoklonalen Antikörpern oder Antikörperfragmenten und autologen Immunglobulinen.

**12.** Verwendung mindestens eines Konjugats erhältlich nach einem oder mehreren der Ansprüche 1 bis 11 zur Herstellung eines Tumordiagnostikums.

**13.** Verwendung mindestens eines Konjugats erhältlich nach einem oder mehreren der Ansprüche 1 bis 11 zur Herstellung eines Tumortherapeutikums.

**14.** Verfahren zur Herstellung eines Tumortherapeutikums, dadurch gekennzeichnet, daß man mindestens ein Konjugat, erhältlich nach einem oder mehreren der Ansprüche 1 bis 11, mit einem physiologisch verträglichen Injektionsvehikel mischt.

**15.** Verfahren zur Herstellung eines Tumordiagnostikums, dadurch gekennzeichnet, daß man mindestens ein Konjugat, erhältlich nach einem oder mehreren der Ansprüche 1 bis 11, mit einem physiologisch verträglichen Injektionsvehikel mischt.

## Claims

### Claims for the following Contracting States : AT, BE, CH, DE, DK, FR, GB, IT, LI, LU, NL, SE

**1.** A conjugate composed of
a) at least one polyalcohol or one derivatized polyalcohol,
b) at least one active agent,
c) at least one linker, and
d) a protein, wherein
the polyalcohol(s) or the derivatized polyalcohol(s) are polyalcohols or derivatized polyalcohols which are not recognized by the defense system of an organism as exogenous, and the protein is a protein which can be taken up by the tumor specifically or non-specifically and is not recognized by the defense system of an organism as exogenous, characterized in that the polyalcohol(s) or the derivatized polyalcohol(s) are a compound of formula I

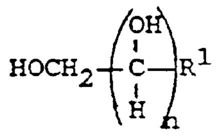

(I)

in which
$R^1$ is $CH_2OH$, CHO or $CH_2NH_2$ and
n is 1 - 10
and in which a

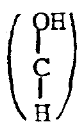

group can be replaced by CO and wherein zero, one or more OH groups can be replaced by $NH_2$, $^{19}F$, $C^{19}F_3$, mono- or poly-$^{19}F$-substituted $C_1$-$C_4$-alkyl or mono-, di-, tri-, tetra- or penta-$^{19}F$-substituted phenyl, or the polyalcohol is glucose, fructose, maltose, sucrose or sorbitol, or the polyalcohol derivative is glucose, fructose, maltose, sucrose or sorbitol, with at least one OH group in these compounds being replaced by $^{19}F$, $C^{19}F3$, mono- or poly-$^{19}F$-substituted $C_1$-$C_4$-alkyl or mono-, di-, tri-, tetra- or penta-$^{19}F$-substituted phenyl.

2. A conjugate according to claim 1, characterized in that the derivatized polyalcohol(s) which is or are not recognized as exogenous by the defense system of an organism, or the linker(s), assume the function of the active agent(s).

3. A conjugate according to claim 1 or 2, characterized in that components a), b), c) and d) are connected together covalently.

4. A conjugate according to one or more of claims 1 to 3, characterized in that component a) is composed of a polyalcohol or a derivatized polyalcohol which is not recognized as exogenous by the defense system of an organism.

5. A conjugate according to one or more of claims 1 to 4, characterized in that component b) is composed of one or two active agents.

6. A conjugate according to one or more of claims 1 to 5, characterized in that component c) is composed of a linker.

7. A conjugate according to one or more of claims 1 to 6, characterized in that the autologous polyalcohol(s) which is or are not recognized as exogenous by the defense system of an organism are selected from the group consisting of
glucose, fructose, maltose, sucrose, sorbitol and glyceraldehyde.

8. A conjugate according to one or more of claims 1 to 7, characterized in that the active agent(s) are selected from
radioactively labeled compounds, a fluorescence label, an NMR label, an ESR label, a photoactive compound, a $^{10}B$- containing compound, a cytostatic.

9. A conjugate according to one or more of claims 1 to 8, characterized in that the active agent(s) are selected from compounds of the formula II

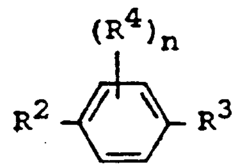

(II)

in which
$R^2$ is $NH_2$, $C_1$-$C_4$-alkylamine or CHO, and
$R^3$ is $NO_2$, $NH_2$, OH or NCS, and
$R^4$ is $^{131}I$, $^{123}I$, $^{19}F$, $C^{19}F3$, OH, H, and
n is 1 to 4.

**10.** A conjugate according to one or more of claims 1 to 9, characterized in that the linker(s) are selected from 2,4-dichloropyrimidine, 4,4′-diisothiocyano-2,2′-stilbenedisolfonic acid, tyramine, benzaldehyde and cyanuric chloride.

**11.** A conjugate according to one or more of claims 1 to 10, characterized in that the protein which is not recognized as exogenous by the defense system of an organism and can be taken up specifically or non-specifically is selected from autologous serum albumin, autologous polyclonal or monoclonal antibodies or antibody fragments and autologous immunoglobulins.

**12.** A process for the preparation of the conjugate according to claim 1, characterized in that chemical bonds are produced between components a) and b) and c) and d).

**13.** A process according to claim 12, characterized in that the chemical bonds are covalent.

**14.** An agent for diagnosing tumors, containing at least one conjugate according to one or more of claims 1 to 11.

**15.** An agent for the therapy of tumors, containing at least one conjugate according to one or more of claims 1 to 11.

**16.** The use of at least one conjugate according to one or more of claims 1 to 11 for the preparation of an agent for diagnosing tumors.

**17.** The use of at least one conjugate according to one or more of claims 1 to 11 for the preparation of an agent for the therapy of tumors.

**18.** A process for the preparation of an agent for the therapy of tumors, characterized in that at least one conjugate according to one or more of claims 1 to 11 is mixed with a physiologically tolerated injection vehicle.

**19.** A process for the preparation of an agent for diagnosing tumors, characterized in that at least one conjugate according to one or more of claims 1 to 11 is mixed with a physiologically tolerated injection vehicle.

**Claims for the following Contracting States : ES, GR**

**1.** A process for the preparation of a conjugate composed of
a) at least one polyalcohol or one derivatized polyalcohol,
b) at least one active agent,
c) at least one linker, and
d) a protein, wherein
the polyalcohol(s) or the derivatized polyalcohol(s) are polyalcohols or derivatized polyalcohols which are not recognized by the defense system of an organism as exogenous, and the protein is a protein which can be taken up by the tumor specifically or non-specifically and is not recognized by the defense system of an organism as exogenous, and in which chemical bonds are produced between components a), b), c) and d), characterized in that the polyalcohol(s) or the derivatized polyalcohol(s) are a compound of formula I

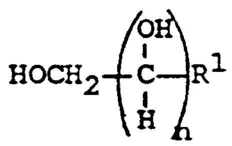

(I)

in which
$R^1$ is $CH_2OH$, CHO or $CH_2NH_2$ and
n is 1 - 10
and in which a

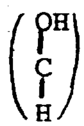

group can be replaced by CO and wherein zero, one or more OH groups can be replaced by $NH_2$, $^{19}F$, $C^{19}F_3$, mono- or poly-$^{19}F$-substituted $C_1$-$C_4$-alkyl or mono-, di-, tri-, tetra- or penta-$^{19}F$-substituted phenyl, or the polyalcohol is glucose, fructose, maltose, sucrose or sorbitol, or the polyalcohol derivative is glucose, fructose, maltose, sucrose or sorbitol, with at least one OH group in these compounds being replaced by $^{19}F$, $C^{19}F3$, mono- or poly-$^{19}F$-substituted $C_1$-$C_4$-alkyl or mono-, di-, tri-, tetra- or penta-$^{19}F$-substituted phenyl.

2. A process according to claim 1, characterized in that the derivatized polyalcohol(s) which is or are not recognized as exogenous by the defense system of an organism, or the linker(s), assume the function of the active agent(s).

3. A process according to claim 1 or 2, characterized in that components a), b), c) and d) are connected together covalently.

4. A process according to one or more of claims 1 to 3, characterized in that component a) is composed of a polyalcohol or a derivatized polyalcohol which is not recognized as exogenous by the defense system of an organism.

5. A process according to one or more of claims 1 to 4, characterized in that component b) is composed of one or two active agents.

6. A process according to one or more of claims 1 to 5, characterized in that component c) is composed of a linker.

7. A process according to one or more of claims 1 to 6, characterized in that the autologous polyalcohol(s) which is or are not recognized as exogenous by the defense system of an organism are selected from the group consisting of

glucose, fructose, maltose, sucrose, sorbitol and glyceraldehyde.

8. A process according to one or more of claims 1 to 7, characterized in that the active agent(s) are selected from

radioactively labeled compounds, a fluorescence label, an NMR label, an ESR label, a photoactive compound, a $^{10}B$-containing compound, a cytostatic.

9. A process according to one or more of claims 1 to 8, characterized in that the active agent(s) are selected from compounds of the formula II

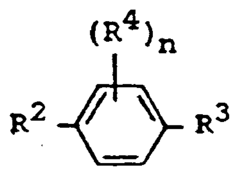

(II)

in which
$R^2$ is $NH_2$, $C_1$-$C_4$-alkylamine or CHO, and
$R^3$ is $NO_2$, $NH_2$, OH or NCS, and
$R^4$ is $^{131}I$, $^{123}I$, $^{19}F$, $C^{19}F_3$, OH, H, and
n is 1 to 4.

**10.** A process according to one or more of claims 1 to 9, characterized in that the linker(s) are selected from 2,4-dichloropyrimidine, 4,4′-diisothiocyano-2,2′-stilbenedisolfonic acid, tyramine, benzaldehyde and cyanuric chloride.

**11.** A process according to one or more of claims 1 to 10, characterized in that the protein which is not recognized as exogenous by the defense system of an organism and can be taken up specifically or non-specifically is selected from autologous serum albumin, autologous polyclonal or monoclonal antibodies or antibody fragments and autologous immunoglobulins.

**12.** The use of at least one conjugate obtainable according to one or more of claims 1 to 11 for the preparation of an agent for diagnosing tumors.

**13.** The use of at least one conjugate obtainable according to one or more of claims 1 to 11 for the preparation of an agent for the therapy of tumors.

**14.** A process for the preparation of an agent for the therapy of tumors, characterized in that at least one conjugate obtainable according to one or more of claims 1 to 11 is mixed with a physiologically tolerated injection vehicle.

**15.** A process for the preparation of an agent for diagnosing tumors, characterized in that at least one conjugate obtainable according to one or more of claims 1 to 11 is mixed with a physiologically tolerated injection vehicle.

## Revendications

**Revendications pour les Etats contractants suivants : AT, BE, CH, DE, DK, FR, GB, IT, LI, LU, NL, SE**

**1.** Conjugué composé de :
a) au moins un polyol ou un polyol dérivé,
b) au moins un agent actif,
c) au moins un segment de liaison, et
c) une protéine,
dans lequel le ou les polyols ou le ou les polyols dérivés sont des polyols ou des polyols dérivés qui ne sont pas reconnus comme étrangers au corps par le système de défense d'un organisme, et la protéine est une protéine qui peut être fixée de manière spécifique ou non spécifique par la tumeur et n'est pas reconnue comme étrangère au corps par le système de défense d'un organisme, caractérisé en ce que le ou les polyols ou le ou les polyols dérivés sont un composé de formule I :

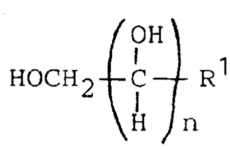

(I)

dans laquelle
$R^1$ signifie $CH_2OH$, CHO ou $CH_2NH_2$, et
n vaut de 1 à 10,
et dans laquelle un groupe

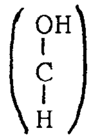

peut être remplacé par CO, et dans laquelle aucun, un ou plusieurs groupes OH peuvent être remplacés par $NH_2$, $^{19}F$, $C^{19}F_3$, un alcoyle en $C_1$-$C_4$ à mono- ou polysubstitution $^{19}F$, ou un phényle à mono-, di-, tri-, tétra- ou penta-substitution $^{19}F$, ou le polyol est le glucose, le fructose, le maltose, le saccharose ou le sorbitol, ou le dérivé de polyol est le glucose, le fructose, le maltose, le saccharose ou le sorbitol, au moins un groupe OH de ces composés étant remplacé par $^{19}F$, $C^{19}F_3$, un alcoyle en $C_1$-$C_4$ à mono- ou polysubstitution $^{19}F$ ou un phényle à mono-, di-, tri-, tétra- ou penta-substitution $^{19}F$.

**2.** Conjugué selon la revendication 1, caractérisé en ce que le ou les polyols non reconnus comme étrangers au corps par le système de défense d'un organisme ou le ou les segments de liaison remplissent là fonction du ou des agents actifs.

**3.** Conjugué selon la revendication 1 ou 2, caractérisé en ce que les composants a), b), c) et d) sont reliés ensemble de manière covalente.

**4.** Conjugué selon une ou plusieurs des revendications 1 à 3, caractérisé en ce que le composant a) est formé d'un polyol ou d'un polyol dérivé qui n'est pas reconnu comme étranger au corps par le système de défense d'un organisme.

**5.** Conjugué selon une ou plusieurs des revendications 1 à 4, caractérisé en ce que le composant b) est formé d'un ou deux agents actifs.

**6.** Conjugué selon une ou plusieurs des revendications 1 à 5, caractérisé en ce que le composant c) est formé d'un segment de liaison.

**7.** Conjugué selon une ou plusieurs des revendications 1 à 6, caractérisé en ce qu'on choisit le ou les polyols autologues non reconnus comme étrangers au corps par le système de défense d'un organisme dans le groupe comprenant le glucose, le fructose, le maltose, le saccharose, le sorbitol et l'aldéhyde de glycérol.

**8.** Conjugué selon une ou plusieurs des revendications 1 à 7, caractérisé en ce qu'on choisit le ou les agents actifs parmi des composés marqués radioactivement, un marqueur fluorescent, un marqueur de RMN, un marqueur de RSE, un composé photo-actif, un composé contenant du $^{10}B$, ou un agent cytostatique.

**9.** Conjugué selon une ou plusieurs des revendications 1 à 8, caractérisé en ce qu'on choisit le ou les agents actifs parmi des composés répondant à la formule II

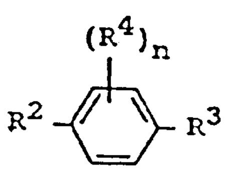

(II)

dans laquelle
$R^2$ signifie $NH_2$, une alcoylamine en $C_1$-$C_4$ ou CHO,
$R^3$ signifie $NO_2$, $NH_2$, OH ou NCS,
$R^4$ signifie $^{131}J$, $^{123}J$, $^{19}F$, $C^{19}F_3$, OH ou H, et
n vaut de 1 à 4.

**10.** Conjugué selon une ou plusieurs des revendications 1 à 9, caractérisé en ce qu'on choisit le ou les segments de liaison parmi la 2,4-dichloropyrimidine, l'acide stilbène-4,4'-diisothiocyano-2,2'-disulfonique, la tyramine, le benzaldéhyde et le chlorure de cyanuryle.

**11.** Conjugué selon une ou plusieurs des revendications 1 à 10, caractérisé en ce que la protéine non reconnue comme étrangère au corps par le système de défense d'un organisme et pouvant être fixée de manière spécifique ou non spécifique est choisie parmi la sérum-albumine autologue, des anticorps ou fragments d'anticorps autologues polyclonaux ou monoclonaux et des immunoglobulines autologues.

**12.** Procédé de préparation du conjugué selon la revendication 1, caractérisé en ce qu'on produit des liaisons chimiques entre les composants a) et b) et c) et d).

**13.** Procédé selon la revendication 12, caractérisé en ce que les liaisons chimiques sont covalentes.

**14.** Agent de diagnostic de tumeurs, contenant au moins un conjugué selon une ou plusieurs des revendications 1 à 11.

**15.** Agent thérapeutique pour tumeurs, contenant au moins un conjugué selon une ou plusieurs des revendications 1 à 11.

**16.** Utilisation d'au moins un conjugué selon une ou plusieurs des revendications 1 à 11 pour la préparation d'un agent de diagnostic de tumeurs.

**17.** Utilisation d'au moins un conjugué selon une ou plusieurs des revendications 1 à 11 pour la préparation d'un agent thérapeutique pour tumeurs.

**18.** Procédé de préparation d'un agent thérapeutique pour tumeurs, caractérisé en ce qu'on mélange au moins un conjugué selon une ou plusieurs des revendications 1 à 11 avec un véhicule pour injection physiologiquement compatible.

**19.** Procédé de préparation d'un agent de diagnostic de tumeurs, caractérisé en ce qu'on mélange au moins un conjugué selon une ou plusieurs des revendications 1 à 11 avec un véhicule pour injection physiologiquement compatible.

**Revendications pour les Etats contractants suivants : ES, GR**

**1.** Procédé de préparation du conjugué composé de:
   a) au moins un polyol ou un polyol dérivé,
   b) au moins un agent actif,
   c) au moins un segment de liaison, et
   c) une protéine,

dans lequel le ou les polyols ou le ou les polyols dérivés sont des polyols ou des polyols dérivés qui ne sont pas reconnus comme étrangers au corps par le système de défense d'un organisme, et la protéine est une protéine qui peut être fixée de manière spécifique ou non spécifique par la tumeur et n'est pas reconnue comme étrangère au corps par le système de défense d' un organisme, et en ce qu'on produit des liaisons chimiques entre les composants a) , b) , c) et d), charactérisé en ce que le ou les polyols ou le ou les polyols dérivés sont un composé de formule I:

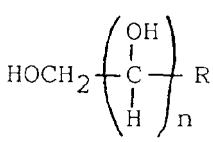

(I)

dans laquelle
$R^1$ signifie $CH_2OH$, CHO ou $CH_2NH_2$, et
n vaut de 1 à 10,
et dans laquelle un groupe

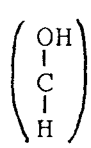

peut être remplacé par CO, et dans laquelle aucun, un ou plusieurs groupes OH peuvent être remplacés par $NH_2$, $^{19}F$, $C^{19}F_3$, un alcoyle en $C_1$-$C_4$ à mono- ou polysubstitution $^{19}F$, ou un phényle à mono-, di-, tri-, tétra- ou penta-substitution $^{19}F$, ou le polyol est le glucose, le fructose, le maltose, le saccharose ou le sorbitol, ou le dérivé de polyol est le glucose, le fructose, le maltose, le saccharose ou le sorbitol, au moins un groupe OH de ces composés étant remplacé par $^{19}F$, $C^{19}F_3$, un alcoyle en $C_1$-$C_4$ à mono- ou polysubstitution $^{19}F$ ou un phényle à mono-, di-, tri-, tétra- ou penta-substitution $^{19}F$.

2. Procédé selon la revendication 1, caractérisé en ce que le ou les polyols non reconnus comme étrangers au corps par le système de défense d'un organisme ou le ou les segments de liaison remplissent la fonction du ou des agents actifs.

3. Procédé selon la revendication 1 ou 2, caractérisé en ce que les composants a), b), c) et d) sont reliés ensemble de manière covalente.

4. Procédé selon une ou plusieurs des revendications 1 à 3, caractérisé en ce que le composant a) est formé d'un polyol ou d'un polyol dérivé qui n'est pas reconnu comme étranger au corps par le système de défense d' un organisme.

5. Procédé selon une ou plusieurs des revendications 1 à 4, caractérisé en ce que le composant b) est formé d' un ou deux agents actifs.

6. Procédé selon une ou plusieurs des revendications 1 à 5, caractérisé en ce que le composant c) est formé d'un segment de liaison.

7. Procédé selon une ou plusieurs des revendications 1 à 6, caractérisé en ce qu'on choisit le ou les polyols autologues non reconnus comme étrangers au corps par le système de défense d'un organisme dans le groupe comprenant le glucose, le fructose, le maltose, le saccharose, le sorbitol et l'aldéhyde de glycérol.

8. Procédé selon une ou plusieurs des revendications 1 à 7, caractérisé en ce qu'on choisit le ou les agents actifs parmi des composés marqués radioactivement, un marqueur fluorescent, un marqueur de RMN,un marqueur de RSE, un composé photo-actif, un composé contenant du $^{10}B$, ou un agent cytostatique.

9. Procédé selon une ou plusieurs des revendications 1 à 8, caractérisé en ce qu'on choisit le ou les agents actifs parmi des composés répondant à la formule II

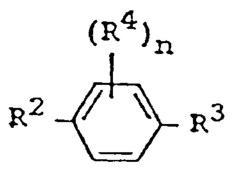

(II)

dans laquelle
$R^2$ signifie $NH_2$, une alcoylamine en $C_1$-$C_4$ ou CHO,
$R^3$ signifie $NO_2$, $NH_2$, OH ou NCS,
$R^4$ signifie $^{131}J$, $^{123}J$, $^{19}F$, $C^{19}F_3$, OH ou H, et
n vaut de 1 à 4.

10. Procédé selon une ou plusieurs des revendications 1 à 9, caractérisé en ce qu'on choisit le ou les segments de liaison parmi la 2,4-dichloropyrimidine, l'acide stilbène-4,4′-diisothiocyano-2,2′-disulfonique, la tyramine, le benzaldéhyde et le chlorure de cyanuryle.

11. Procédé selon une ou plusieurs des revendications 1 à 10, caractérisé en ce que la protéine non reconnue comme étrangère au corps par le système de défense d'un organisme et pouvant être fixée de manière spécifique ou non spécifique est choisie parmi la sérum-albumine autologue, des anticorps ou fragments d'anticorps autologues polyclonaux ou monoclonaux et des immunoglobulines autologues.

12. Utilisation d'au moins un conjugué selon une ou plusieurs des revendications 1 à 11 pour la préparation d'un agent de diagnostic de tumeurs.

13. Utilisation d'au moins un conjugué selon une ou plusieurs des revendications 1 à 11 pour la préparation d'un agent thérapeutique pour tumeurs.

14. Procédé de préparation d'un agent thérapeutique pour tumeurs, caractérisé en ce qu'on mélange au moins un conjugué selon une ou plusieurs des revendications 1 à 11 avec un véhicule pour injection physiologiquement compatible.

15. Procédé de préparation d'un agent de diagnostic de tumeurs, caractérisé en ce qu'on mélange au moins un conjugué selon une ou plusieurs des revendications 1 à 11 avec un véhicule pour injection physiologiquement compatible.

131I-TDS-RSA
Aktivitätsverteilung
in BD IX Ratten mit Overialcarcinom

**Fig. 1**

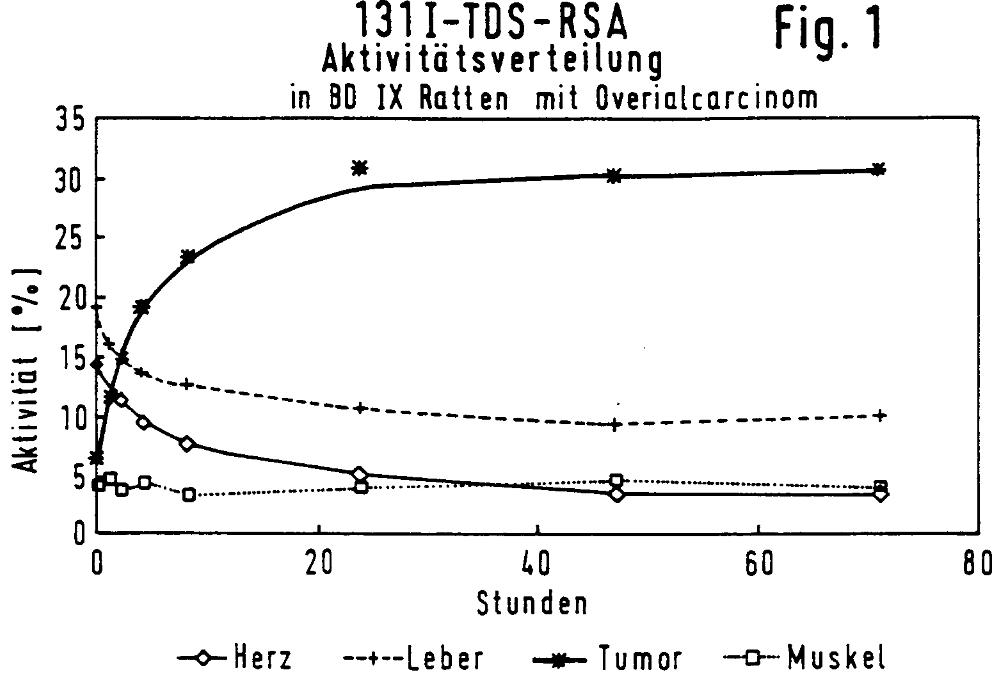


131I-TDS-IgG
Aktivitätsverteilung
in BD IX Ratten mit Overialcarcinom

**Fig. 2**